# EUROPEAN PATENT SPECIFICATION

(11) **EP 2 582 841 B1**
(45) Date of publication and mention of the grant of the patent: **01.03.2017**
(21) Application number: 11730603.5
(22) Date of filing: 20.06.2011
(51) Int. Cl.: C12Q 1/68

(54) **METHODS FOR HAPLOTYPING SINGLE CELLS**
VERFAHREN ZUR HAPLOTYPBESTIMMUNG VON EINZELNEN ZELLEN
PROCÉDÉS D'HAPLOTYPAGE DE CELLULES ISOLÉES

(30) Priority: 18.06.2010 GB 201010232
(43) Date of publication of application: 24.04.2013
(73) Proprietor: Katholieke Universiteit Leuven, 3000 Leuven (BE)
(72) Inventor: VERMEESCH, Joris, B-3020 Veltem-Beisem (BE); VOET, Thierry, B-3001 Heverlee (BE); ZAMANI ESTEKI, Masoud, B-3000 Leuven (BE)
(74) Representative: LC Patents
(86) International application number: PCT/EP2011/060211
(87) International publication number: WO 2011/157846

(56) References cited:
- WO-A1-2009/105531
- SHARON R. BROWNING ET AL: "Haplotype phasing: existing methods and new developments", NATURE REVIEWS GENETICS, vol. 12, no. 10, 1 January 2011 (2011-01-01), pages 703-714, XP55008581, ISSN: 1471-0056, DOI: 10.1038/nrg3054
- ABECASIS GONCALO R ET AL: "Merlin: Rapid analysis of dense genetic maps using sparse gene flow trees", NATURE GENETICS, NATURE PUBLISHING GROUP, NEW YORK, US, vol. 30, no. 1, 1 January 2002 (2002-01-01), pages 97-101, XP002334050, ISSN: 1061-4036, DOI: 10.1038/NG786 cited in the application
- AIDA M. ANDRÉS ET AL: "Understanding the accuracy of statistical haplotype inference with sequence data of known phase", GENETIC EPIDEMIOLOGY, vol. 31, no. 7, 1 November 2007 (2007-11-01), pages 659-671, XP55008582, ISSN: 0741-0395, DOI: 10.1002/gepi.20185
- A. H. HANDYSIDE ET AL: "Karyomapping: a universal method for genome wide analysis of genetic disease based on mapping crossovers between parental haplotypes", JOURNAL OF MEDICAL GENETICS, vol. 47, no. 10, 25 October 2009 (2009-10-25), pages 651-658, XP055146321, ISSN: 0022-2593, DOI: 10.1136/jmg.2009.069971
- SIBLEY G ET AL: "A sliding window filter for incremental SLAM", LECTURE NOTES IN ELECTRICAL ENGINEERING - UNIFYING PERSPECTIVES IN COMPUTATIONAL AND ROBOT VISION2008SPRINGER VERLAGDEU, vol. 8, 2008, pages 103-112, XP009186047, ISSN: 1876-1100

## Description

### 1. Field of the invention

The present invention relates to methods for genotyping single or few (human) cells using high resolution genome-wide genetic polymorphism typing platforms, e.g. single nucleotide polymorphism (SNP)-arrays or massively parallel DNA-sequencers, for subsequent haplotype reconstruction, more particularly to methods for single- and dual-cell haplotyping. The developed generic methods have immediate applicative value for (1) preimplantation genetic diagnosis (PGD) of *in vitro* fertilized human embryos in the clinic, (2) animal breeding programs by enabling selection of embryos for multiple (quantitative trait) loci in a single experiment, (3) genetic studies of heterogeneous tissues that consist of cells with different allelic constitutions (e.g. tumors), as well as (4) all genetic studies requiring genetic polymorphism typing (such as SNP typing or genetic variant detection by DNA-sequencing) or haplotyping data in general.

### 2. Background art

A haplotype represents the succession of alleles in synteny and can be determined *in silico* from observed genotype data by statistical and computational methods (Gao et al. 2009). The inference of haplotypes has proven invaluable not only to study the relationship between an inherited disease and its genetic cause (Altshuler et al. 2008), but also to unravel the genetic architecture of Mendelian and complex traits like quantitative trait loci (Goddard and Hayes 2009), to study evolution and population history as well as meiotic and mitotic recombination processes. In addition, computation of the sequence of syntenic alleles can be used to identify genotyping errors by looking for successive homologous recombination sites in a limited locus (Abecasis et al. 2002) and to infer missing genotype calls as well. Until recently, genotypes were mainly determined by analyzing one or a few polymorphic markers per reaction. The recent development of several genome-wide high-throughput approaches for marker analyses have enabled to type thousands to millions of polymorphic markers in a single experiment. For instance, microarrays with oligonucleotides targeting single nucleotide polymorphisms over the entire genome have enabled massively parallel SNP re-sequencing and have accelerated the identification of genetic variants underlying specific traits or diseases in both humans and animals mainly through linkage analyses and genome-wide association studies (Altshuler et al. 2008; Goddard and Hayes 2009; Frazer et al. 2009). In addition, SNP-typing has been used to identify regions of copy neutral loss of heterozygosity (CN-LOH) on a genome-wide scale in a large series of acute myeloid leukaemia samples, which so far has not been possible through any other technology. The development of massively parallel sequencing techniques enabled the detection of the full spectrum of genetic variants and polymorphisms, including e.g. SNPs, insertions-deletions (indels), copy number polymorphisms (CNVs) as well as balanced and unbalanced structural genetic variants in a single experiment. These sequencing technologies for genotyping and haplotyping further boosted biomedical research and enabled novel clinical applications.

Most genotyping and haplotyping methods however make use of large amounts of DNA and, hence, use DNA that is extracted from a large number of cells of the organism. In many applications though, only a few or even a single cell is available for analysis. In the study of meiotic recombination processes often single haploid sperm cells are genotyped. In preimplantation genetic diagnosis, PCR-based 'simple tandem repeat' (STR) marker analysis is often used to (haplo)type a small locus in a single blastomere (Renwick et al. 2006). In addition, the analysis of a mixture of cells obscures the presence of different subpopulations or cells with a different allelic constitution. For example, cancers acquire cumulative changes and tumor DNA-samples are often contaminated with DNA from normal cells as well. As a consequence analyzing such heterogeneous DNA-samples may lead to loss of valuable genetic information.

The developed single- and dual-cell haplotyping methods of the present invention allow monitoring the inheritance of loci in a generic and genome-wide fashion by genetic polymorphism typing, e.g. SNP-typing, of DNA-samples derived from single cells. Hence it has the capacity to improve and standardize current preimplantation genetic diagnosis (PGD). PGD is an optional method for couples that wish to circumvent the transmission of heritable diseases to their offspring. It is underpinned by genetic testing of usually a single or a pair of blastomeres that are biopsied from an *in vitro* fertilized (IVF) embryo on day 3 after fertilization. Embryos without the specific mutation can subsequently be transferred to the woman's uterus on day 4 or 5 after fertilization. Currently, monogenic disorders in pre-implantation embryos are generally diagnosed using PCR amplification of the mutated allele and one or two linked simple tandem repeat (STR) markers in a multiplex reaction directly on the single-cell DNA or on multiple displacement amplified (MDA) single-cell DNA. However, this approach requires time-consuming designs of family-specific single-cell multiplex PCR reactions that are optimized for minimum allele drop out (ADO), which represents the random failure of amplification of one or multiple alleles at a locus, to avoid misdiagnosis. Furthermore, DNA and amplicon contamination has to be carefully monitored, hence the inclusion of the linked polymorphic markers in the reaction. To get round these difficulties, Renwick et al. (2006) developed preimplantation genetic haplotyping (PGH) which is based on testing family members for multiple polymorphic markers that locate in the close vicinity of a genetic mutation enabling the detection of high-risk and low-risk haplotypes in MDA-amplified single-cell DNA. By including multiple markers in the tests, problems with uninformative markers for specific families, ADO and recombination between a mutation and a linked marker are overcome. However, this technology still requires the development of multiplex PCRs for each locus to be tested.

These drawbacks are defeated by our single- and dual-cell haplotyping methods of the present invention as it uses information from thousands of polymorphic markers spread across the genome allowing to follow accurately the segregation of any segment of the genome within a pedigree.

Genome-wide genotyping and haplotyping methods for single cells have been developed previously. US20040259100 provides a method for genome-wide genotyping using a SNP array. However, the method is prone to genotyping errors when single-cell genomes are used. Those errors can, for example, result from single-cell whole genome amplification artifacts like ADO and preferential amplification (PA) of alleles. Single-cell genotyping errors when using SNP arrays were also detected by Iwamoto et al. (2007). However, the use of erroneous single-cell genotypes for phasing leads to false single-cell haplotype estimations. A method to circumvent genotyping errors when determining the karyotype is provided by US2008318235. This method involves calculating the statistical probabilities of double cross-overs between two SNP alleles.

A drawback of the genome-wide haplotyping method of Handyside et al. (2009), when compared to the present invention, is that it does not account for false-allelic assignments of haplotype blocks, wherein blocks are defined as a certain amount of adjacent SNPs with the same allelic allocation. These false-allelic assignments most likely arise from single-cell genotyping errors that can for instance result from single-cell whole genome amplification artifacts like those described hereinbefore. In this case, the major resulting error is that heterozygous SNPs are typed homozygous following ADO or PA. Although Handyside et al. (2009) in part solves this problem by using only the heterozygous SNPs called within single-cell genotypes, this approach does not correct for allele drop in (Johnson et al. 2010) and algorithmic genotyping errors in single-cell genotypes as presented in the present invention.

A partial solution to this problem is given by the single-cell haplotyping method of WO2009105531. In this method, a DNA sample of one or more cells is divided into a plurality of fractions, after which the DNA sample in each fraction is individually genotyped. Based on these genotypes, the haplotype of the one or more cells is constructed. Nonetheless, dividing the DNA sample into a plurality of fractions and genotyping each sample individually is a cumbersome and expensive procedure.

Our single- and dual-cell haplotyping methods of the present invention are generic thereby for instance circumventing labor-intensive designs of family- and locus-specific multiplex PCR reactions in PGD approaches. In addition, the technology of the present invention may not only outperform PCR-based methods to identify the disease allele but also current FISH-based approaches in PGD to select embryos suitable for uterine transfer. FISH is applied to select against embryos with unbalanced translocations that result from couples in which one of the parents carries a balanced translocation. FISH probes have to be developed and validated for every different translocation. In addition, most FISH approaches used for PGD are unable to distinguish those embryos that carry a balanced translocation from those that do not carry the translocation. Thus, a number of embryos suitable for uterine transfer on the basis of FISH still carry the translocated chromosomes. Using the method of the present invention, embryos that carry unbalanced as well as balanced translocations can be identified. Furthermore, for X-linked recessive disorders female embryos are often selected for by FISH. However, half of the male embryos disapproved for transfer are free of the disease-causing allele, while half of the selected female embryos still carry the mutation. In general, knowing the haplotype that carries a genetic aberration enables to sort affected from non-affected embryos in either sex using the method of the present invention.

The single- and dual-cell haplotyping method of the present invention in addition broadens the perspective of embryo selection from single Mendelian traits to all phenotypic aspects that are associated with genetic polymorphism information. Currently, postnatal genome-wide SNP typing is offered to enable disease risk determination for a series of disorders. Although the applicative value of such genomic-phenotypic screens to human embryos is limited, it has huge potential for quantitative trait loci (QTL)-selection in (large) animal embryos. The majority of important 'performance' traits within livestock and other (large) animals are underpinned by complex inheritance patterns of multiple quantitative trait loci thus requiring genome-wide marker screens to select the best animal (Goddard and Hayes 2009). Currently, marker-assisted selection uses validated markers in linkage disequilibrium with a Mendelian or quantitative trait locus to select new-born animals. However, due to the limited number of QTLs that are tagged by markers with well-validated associations, its ability to predict breeding values is limited. A method that does not require a complete understanding of complex traits has been developed to predict the genetic merit within an animal by using markers spread over the entire genome that have been typed in a reference population (Meuwissen et al. 2001). This genomic selection procedure allows by genome-wide SNP-typing to determine a genomic breeding value that correlates with the true breeding value for a specific animal and is thus expected to have an enormous impact on the speed of breeding programs. The single/dual-cell genotyping and haplotyping methods of the present invention enable to start this selection process at the embryonic level thus accelerating breeding programs even more by saving unnecessary lengthy periods of gestation.

In addition, this novel single/dual-cell genotyping/haplotyping method of the present invention allows further characterization of drivers of haplotype diversity, primarily meiotic homologous recombination, but also mitotic recombination processes that may occur at elevated frequencies during tumorigenesis. Inter- as well as intra-chromosomal rearrangements in somatic cells alter the sequence of syntenic alleles leading to the potential activation of proto-oncogenes and inactivation of tumor suppressor genes. Hence, such recombinations may initiate tumorigenesis, but may also contribute to tumor progression. Due to this chromosome instability cells within a tumor are heterogeneous and in addition tumor biopsies are contaminated with normal somatic cells. The methods of the present invention allow to gain more insight in tumor development and recombination processes.

### 3. Summary of the invention

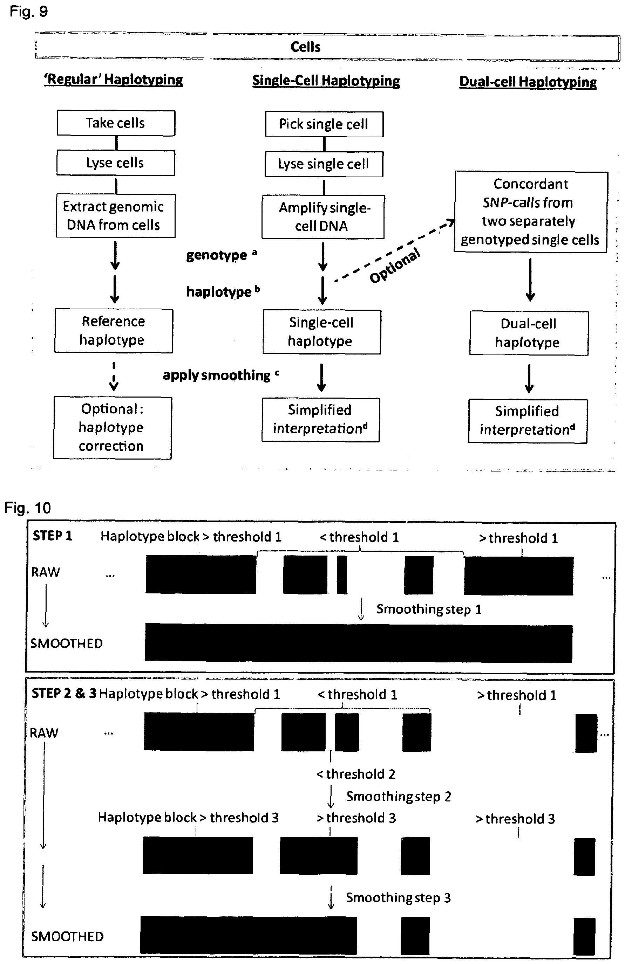

The method of the present invention for single cell or dual-cell haplotyping comprises preferably the following steps (see also flow-chart in Fig. 9):
1. Pick a single cell;
2. Lyse the single cell;
3. Amplify the single cell DNA e.g. by using multiple displacement amplification (MDA) technology;
4. Massively parallel (genome-wide) genetic polymorphism typing (=genotyping) of the amplified single cell DNA; in this step the massively parallel genetic polymorphism typing can e.g. be performed by hybridizing labeled amplified single cell DNA onto an Affymetrix high resolution SNP-array and subsequently using the Dynamic Model algorithm (Di et al. 2005) for interpreting Affymetrix GeneChip SNP-probe intensities;
5. Optional step: select only the heterozygous genetic polymorphisms from the computed single-cell genotypes for subsequent haplotype reconstruction;
6. Optional step: generate a virtual genotype consisting of genetic polymorphisms concordant between two separately genotyped single cells;
7. Reconstruct the haplotype of the single cell's genotype (= single-cell haplotyping; with or without optional step 5, but without optional step 6) or the virtual dual-cell genotype (from optional step 6 = dual cell haplotyping): in this step the haplotyping can be performed using e.g. MERLIN software;
8. Simplify interpretation of the generated single- and/or dual-cell 'raw' haplotypes obtained in the reconstruction by reassigning the allelic allocation of one or more genetic polymorphisms and/or intermediate regions, based on the information of neighboring genetic polymorphisms.

In a preferred embodiment the reassigning of the allelic allocation of one or more genetic polymorphisms and/or intermediate regions is performed by reassigning the allelic allocation of haplotype blocks smaller than a certain amount of genetic polymorphisms (= threshold).

In another preferred embodiment the reassigning of the allelic allocation of haplotype blocks smaller than a certain amount of genetic polymorphisms (= threshold) is done by making use of the grandparental origin of the first haplotype blocks to the left and right of the small haplotype block which are larger than the threshold.

In a preferred embodiment of the present invention the reassigning is performed as follows: haplotype blocks which are smaller, in amount of genetic polymorphisms, than a certain threshold T₁ are assigned to the first haplotype block on the left or right side of the haplotype block larger than the threshold T₁ only if these two haplotype blocks are assigned to the same grandparent. In a specific embodiment the genetic polymorphisms used in the methods according to the present invention, as described hereinbefore and hereinafter, consists of massively parallel (genome-wide) SNP-typing. In said embodiment the threshold value T₁ is equal to 200 SNPs.

In another preferred embodiment more than one cycle of the reassigning procedure described above is performed with different threshold values for T₁.

Furthermore, in one embodiment of the present invention the grandparental origin of the haplotype blocks smaller than the threshold T₁ and located at the p- or q-terminus of a chromosome are adjusted to the first haplotype block larger than the threshold T₁ if the p- or q-terminal haplotype blocks below the threshold T₁ are collectively smaller than a threshold value T_{p-q}. Again, in said embodiment wherein the genetic polymorphism consists of SNP-typing the threshold value T_{p-q} is set to 500 SNPs.

In one preferred embodiment of the present invention a three cycle procedure is performed on the raw haplotype data obtained from the reconstruction:
- In a first cycle haplotype blocks which are smaller (in amount of genetic polymorphisms) than a certain threshold T₁ are assigned to the first haplotype block on the left or right side of the haplotype block larger than the threshold T₁ if these haplotype blocks are assigned to the same grandparent. If this is not the case then no re-assignment is done.
- In a second cycle, performed on the haplotype blocks resulting from the first cycle, the haplotype blocks which are smaller (in amount of genetic polymorphisms) than a certain threshold T₂ are assigned to the first haplotype block on the left or right side of the haplotype block larger than the threshold T₂ if these haplotype blocks are assigned to the same grandparent. If this is not the case then no re-assignment is done.
- In a third cycle, performed on the haplotype blocks resulting from the second cycle haplotype blocks which are smaller (in amount of genetic polymorphisms) than a certain threshold T₃ are assigned to the first haplotype block on the left or right side of the haplotype block larger than the threshold T₃ if these haplotype blocks are assigned to the same grandparent. If this is not the case then no re-assignment is done.

Depending on the single-cell genome amplification method, the type of genetic polymorphism typing platform used, the genotyping algorithm used, the type of informative genetic polymorphisms used (e.g. using only the heterozygous genetic polymorphisms from resulting raw single-cell genotypes) T1, T2 and T3 can vary. In a preferred embodiment of the present invention, T2 is smaller than T1 and T3, and T1 was equal to T3.

In yet another embodiment of the present invention, in the first and last cycle the grandparental origin of the resulting haplotype blocks smaller than the threshold (T₁ and T₃ respectively) located at the p- or q-terminus of a chromosome are adjusted to the first haplotype block larger than the threshold (T₁ and T₃ respectively) if the p- or q-terminal haplotype blocks below the threshold (T₁ and T₃ respectively) are collectively smaller than a threshold value T_{p-q}. In one embodiment of the present invention the treshhold value T_{p-q} is set to 500 SNPs.

In a preferred alternative embodiment of the present invention the reassigning the allelic allocation of one or more genetic polymorphisms and/or intermediate regions (step 8 alternative) is based on a digital image signal-processing technique to reduce noise. In a preferred embodiment of the present invention a "1 D median filter" (Pratt 1978) is applied in this non-linear smoothing algorithm. The technique relies on a sliding window (expressed in amount of genetic polymorphisms) and the adjustment of allelic assignments (expressed in binary format) within each window. The main idea of the median filter is to run through the binary signal entry by entry, replacing each entry with the median of neighboring entries. In one embodiment of the present invention the window comprises 450 SNPs for chromosome 1 when Affymetrix 250K NspI SNP-arrays were used for single-cell genotyping. The windows for the other chromosomes are defined by the formula: chromosome-specific window = (number of SNPs in the specific chromosome) / (number of SNPs in chromosome 1) * window size for chromosome 1). Alternatives to the presented window sizes for each chromosome are possible as well.

In an optional step of the present invention: the algorithm compares the resulting smoothed haplotype blocks with the original non-smoothed haplotype blocks and determines whether the majority of the genetic polymorphisms in the original non-smoothed haplotype blocks are assigned to the same allele as in the smoothed haplotype block. If this is not the case, the non-smoothed haplotype may be preferred for further interpretation.

In a preferred embodiment of the present invention (step 9): single-cell and/or dual-cell haplotypes that are computed and smoothed by different approaches are integrated for interpretation. E.g., from a single-cell genotype both all genetic polymorphisms and heterozygous genetic polymorphisms are selected for independent haplotype reconstruction, and each resulting 'raw' single-cell haplotype is subsequently smoothed by both methods (described in 'step 8' and 'step 8 alternative'). Finally, all resulting haplotypes are integrated for concordant regions and interpretation.

In a preferred embodiment of the present invention: single-cell and / or dual-cell haplotypes are also integrated with single-cell DNA copy number profiles determined by existing methods (Vanneste et al. 2009) for reliable single-cell genotype and haplotype interpretation. Aneuploidies of whole or segments of chromosomes can produce false diploid genetic polymorphism calls (produced by the genotyping algorithms in step 4) and may lead to false haplotype predictions.

In a preferred embodiment of the present invention the method for single cell haplotyping comprising the steps of
a. Picking a single cell;
b. Lysing said single cell;
c. Amplifying said single cell DNA;
d. Massively parallel (genome-wide) genetic polymorphism typing (=genotyping) of said amplified single cell DNA;
e. Reconstructing the haplotype of the single cell's genotype using all genetic polymorphisms or a selection thereof (e.g. only heterozygous genetic polymorphisms of a single-cell genotype).
f. Reassigning the allelic allocation of one or more genetic polymorphisms and/or intermediate regions, based on the information of neighboring genetic polymorphisms.

In another preferred embodiment the reassigning of the allelic allocation of one or more genetic polymorphisms and/or intermediate regions is performed by reassigning the allelic allocation of haplotype blocks smaller than a certain amount of genetic polymorphisms.

In yet another preferred embodiment the reassigning of the allelic allocation of haplotype blocks smaller than a certain amount of genetic polymorphisms makes use of the grandparental origin of haplotype blocks larger than said amount of genetic polymorphisms.

In another preferred embodiment the reassigning of the allelic allocation of haplotype blocks smaller than a certain amount of genetic polymorphisms (threshold) is done by making use of the grandparental origin of the first left and right flanking (with respect to the small haplotype block) haplotype blocks which are larger than the threshold.

In a preferred embodiment haplotype blocks which are smaller, in amount of genetic polymorphisms, than a certain threshold T₁ are assigned to the first haplotype block on the left or right side of the haplotype block larger than the threshold T₁ only if these two haplotype blocks are assigned to the same grandparent.

In an alternative preferred embodiment, the reassigning the allelic allocation of one or more genetic polymorphisms and/or intermediate regions is based on a digital image signal-processing technique to reduce noise. In a preferred embodiment of the present invention a "1 D median filter" is applied.

In another preferred embodiment of the present invention: single-cell haplotypes that are computed and smoothed by different approaches are integrated for interpretation. In a preferred embodiment of the present invention: single-cell haplotypes are also integrated with single-cell DNA copy number profiles determined by existing methods (Vanneste et al. 2009) for reliable single-cell genotype and haplotype interpretation.

Another aspect of the invention concerns a method for dual cell haplotyping, said method comprising the steps of
a. Picking two cells;
b. Lysing said two cells;
c. For each cell, amplifying the single cell DNA;
d. For each cell, massively parallel (genome-wide) genetic polymorphism typing (=genotyping) of the amplified single cell DNA;
e. Generating a virtual genotype consisting of genetic polymorphism calls concordant between the two separately genotyped single cells;
f. Reconstructing the haplotype of said virtual genotype (or a selection of said virtual genotype)
g. Reassigning the allelic allocation of one or more genetic polymorphisms and/or intermediate regions, based on the information of neighboring genetic polymorphisms

In a preferred embodiment the reassigning of the allelic allocation of one or more genetic polymorphisms and/or intermediate regions is performed by reassigning the allelic allocation of haplotype blocks smaller than a certain amount of genetic polymorphisms.

In another preferred embodiment the reassigning of the allelic allocation of haplotype blocks smaller than a certain amount of genetic polymorphisms makes use of the grandparental origin of haplotype blocks larger than said amount of genetic polymorphisms.

In yet another preferred embodiment the reassigning of the allelic allocation of haplotype blocks smaller than a certain amount of genetic polymorphisms (threshold) is done by making use of the grandparental origin of the first left and right flanking (with respect to the small haplotype block) haplotype blocks which are larger than the threshold.

In a preferred embodiment haplotype blocks which are smaller, in amount of genetic polymorphisms, than a certain threshold T₁ are assigned to the first haplotype block on the left or right side of the haplotype block larger than the threshold T₁ only if these two haplotype blocks are assigned to the same grandparent.

In an alternative preferred embodiment, the reassigning the allelic allocation of one or more genetic polymorphisms and/or intermediate regions is based on a digital image signal-processing technique to reduce noise. In a preferred embodiment of the present invention a "1 D median filter" is applied.

In another preferred embodiment of the present invention: dual-cell haplotypes that are computed and smoothed by different approaches are integrated for interpretation. In a preferred embodiment of the present invention: dual-cell haplotypes are also integrated with single-cell DNA copy number profiles determined by existing methods (Vanneste et al. 2009) for reliable genotype and haplotype interpretation.

Another aspect of the present invention comprises a method for improving the interpretation of single cell or dual cell derived haplotypes comprising processing of the haplotype blocks by reassigning the allelic allocation of haplotype blocks smaller than a certain amount of genetic polymorphisms.

In a preferred embodiment the reassigning of the allelic allocation of haplotype blocks smaller than a certain amount of genetic polymorphisms makes use of the grandparental origin of haplotype blocks larger than said amount of genetic polymorphisms.

In another preferred embodiment the reassigning of the allelic allocation of haplotype blocks smaller than a certain amount of genetic polymorphisms (threshold) is done by making use of the grandparental origin of the first left and right flanking (with respect to the small haplotype block) haplotype blocks which are larger than the threshold.

In yet another preferred embodiment haplotype blocks which are smaller, in amount of genetic polymorphisms, than a certain threshold T₁ are assigned to the first haplotype block on the left or right side of the haplotype block larger than the threshold T₁ only if these two haplotype blocks are assigned to the same grandparent.

In an alternative preferred embodiment the reassigning of the allelic allocation of one or more genetic polymorphisms and/or neighboring regions from single-cell or dual-cell raw haplotype blocks uses a digital image processing technique for improving the interpretation of single-cell or dual-cell derived haplotypes. In a preferred embodiment of the present invention a "1 D median filter" is applied. In one embodiment of the present invention the window used by the median filter processing technique comprises 450 SNPs for chromosome 1. The windows for the other chromosomes are defined by the formula: chromosome-specific window = (number of SNPs in the specific chromosome) / (number of SNPs in chromosome 1) * window size for chromosome 1).

In another preferred embodiment of the present invention: single-cell and/or dual-cell haplotypes that are computed and smoothed by different approaches are integrated for haplotype interpretation. In a preferred embodiment of the present invention: single-cell and/or dual-cell haplotypes are also integrated with single-cell DNA copy number profiles determined by existing methods (Vanneste et al. 2009) for reliable genotype and haplotype interpretation.

### 4. Detailed description of the invention:

### Brief description of the drawings:

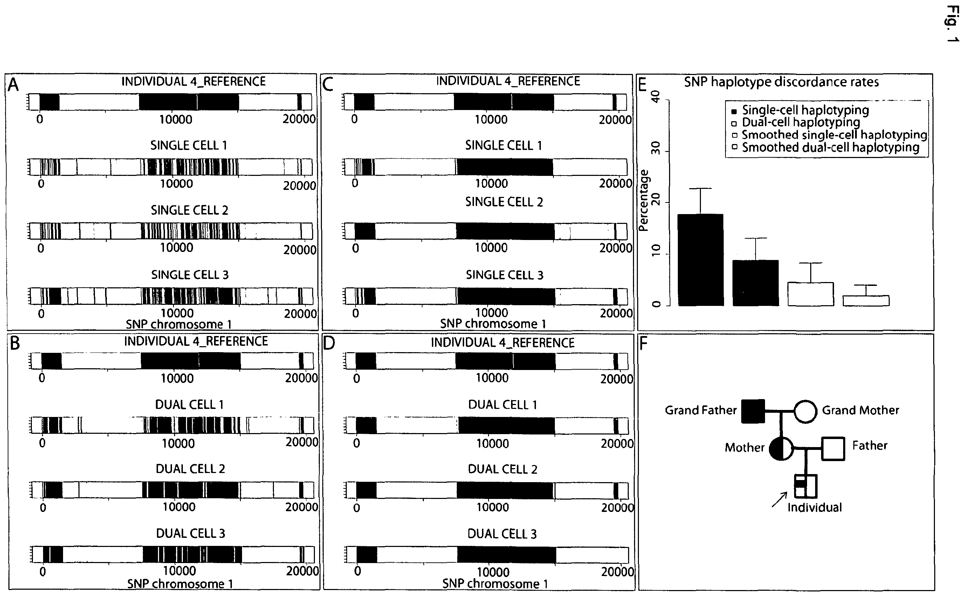

**Fig. 1****: Single- and dual-cell haplotyping, and smoothing of the haplotypes.** (Fig. 1 A-D) Haplotypes of the maternally inherited chromosome 1 from one individual (indicated with an arrow in the pedigree shown in Fig.1 (F). Alleles that trace back to the grandmaternal and grandpaternal chromosomes are indicated in light grey and dark grey respectively. The top lane in each panel represents the reference allelic sequence derived from genomic DNA isolated from multiple cells of this individual. Fig. 1 (A) Single lymphoblastoid-cell derived haplotypes of this individual. The three lower lanes show the 'raw' chromosome 1 haplotypes derived from three independent lymphoblastoid cells respectively. Fig 1. (B) Dual lymphoblastoid-cell derived haplotypes. The three lower lanes show the haplotypes derived from three different virtual genotypes respectively. The virtual genotypes consist of concordant SNP-calls of two single lymphoblastoid cells. Fig 1. (C) Smoothed single-cell haplotypes. The three lower lanes show the smoothed chromosome 1 haplotypes derived from three independent lymphoblastoid cells respectively (shown in Fig. 1. A). Fig. 1. (D) Smoothed results of the haplotypes shown in Fig. 1 (B). Fig. 1 (E) Percentage of autosomal SNPs in haplotype discordance with the reference allelic sequence over the entire genome. Black: single-cell haplotyping, dark grey: dual-cell haplotyping, light grey: smoothing of single-cell haplotypes, white: smoothing of dual-cell haplotypes. The depicted values are averages (%) over all 12 lymphoblastoid analyses (± Standard Deviation %). Fig 1. (F) Pedigree structure of the individual. Alleles that trace back to the grandmaternal and grandpaternal chromosomes of the mother are indicated in light grey and dark grey respectively.

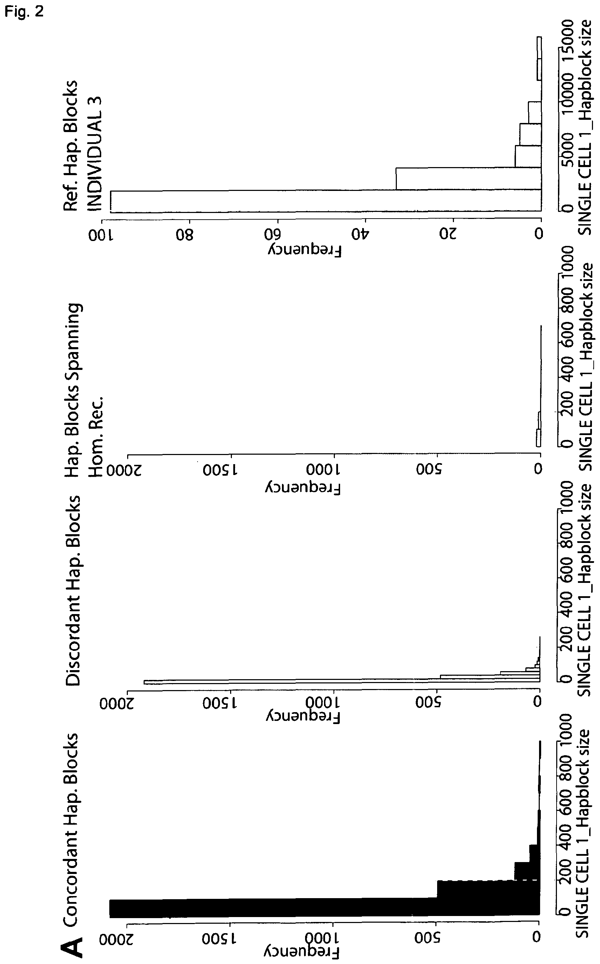

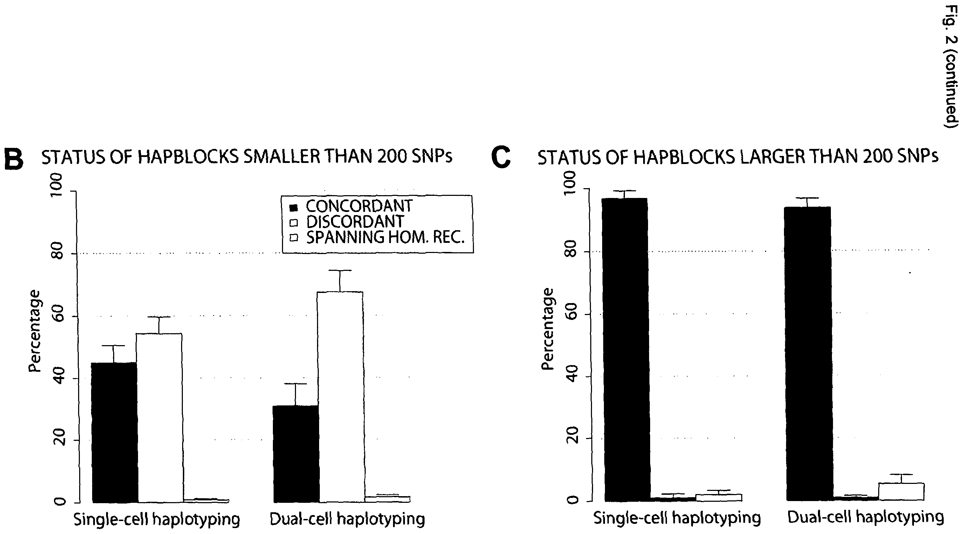

**Fig. 2****: Size distribution of single- and dual-cell derived haplotype blocks.** Fig. 2 (A) Histograms of single-cell derived haplotype block sizes that are concordant (black), discordant (light grey) and spanning a homologous recombination site (white) when compared to the reference allelic sequence (dark grey). The depicted haplotype block sizes are derived from one lymphoblastoid cell analysis. The cutoff of 200 SNPs is indicated in a grey dashed line. Fig. 2 (B) Percentage of single- and dual-cell derived haplotype blocks (left and right bars respectively) that are smaller than 200 SNPs and concordant (black), discordant (light grey) or spanning a homologous recombination site (white) when compared to the reference haplotype. The depicted values are averages (%) over all 12 single- and dual-lymphoblastoid cell analyses (± Standard Deviation %). Fig. 2 (C) Percentage of single- and dual-cell derived haplotype blocks (left and right bars respectively) that are larger than 200 SNPs and concordant (black), discordant (light grey) or spanning a homologous recombination site (white) when compared to the reference haplotype. The depicted values are averages over all 12 single- and dual-lymphoblastoid cell analyses (± Standard Deviation %).

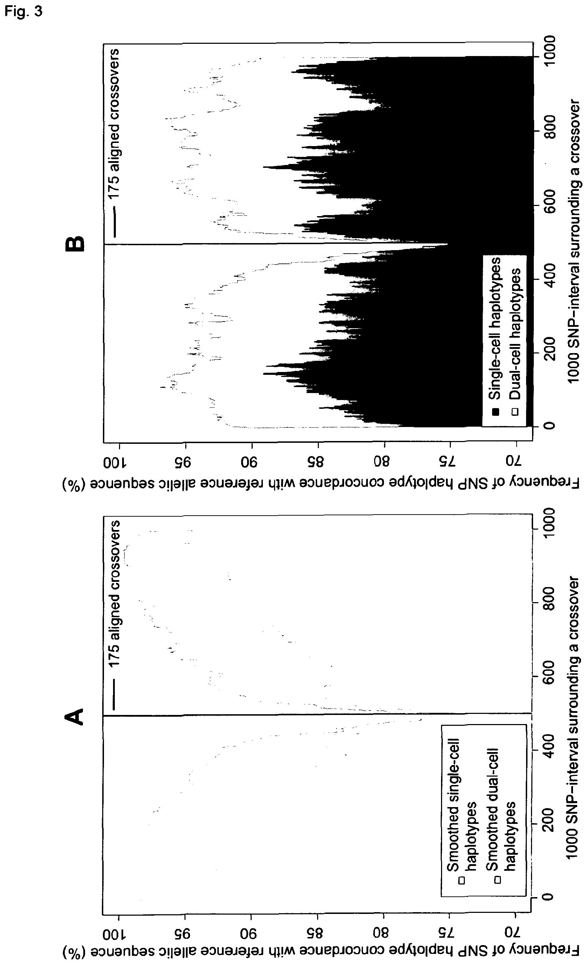

**Fig. 3****: Alignment of single- and dual-cell derived haplotypes at 175 homologous recombination sites.** Single- and dual-cell derived haplotypes, both smoothed and nonsmoothed, were aligned at 175 homologous recombination sites identified in the four individuals. Frequencies (%) of crossover-flanking SNPs that are inferred to the correct haplotype according to the reference allelic sequence are depicted. The line (in the middle of each panel) indicates the pooled 175 crossover sites from the reference allelic sequences. SNP haplotype concordance frequencies were computed for 500 SNPs upstream and downstream of the homologous recombination sites. Fig. 3 (A) Results obtained with smoothed single- and dual-cell derived haplotypes are depicted. Fig. 3 (B) Results obtained with 'raw' single- and dual-cell derived haplotypes are shown.

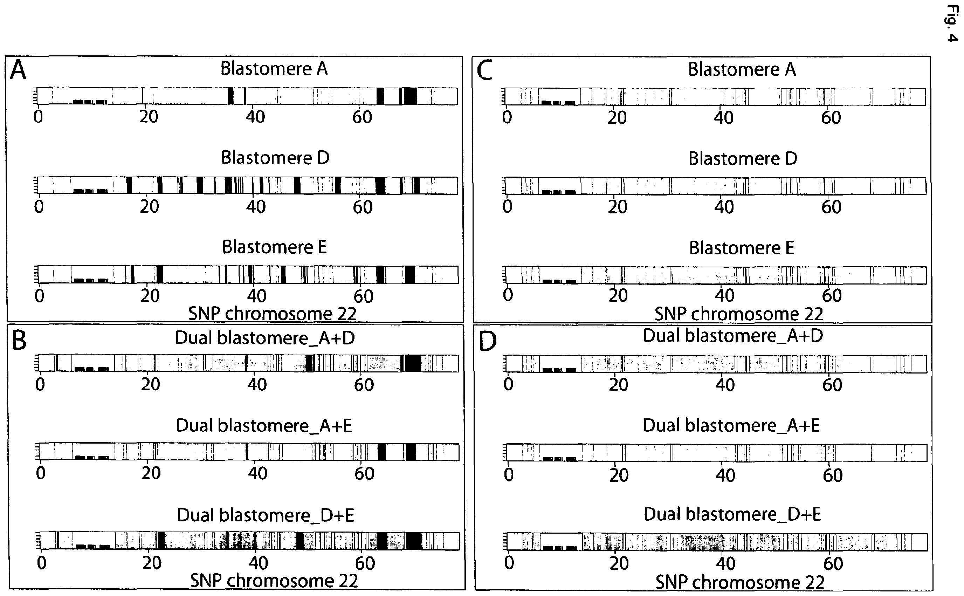

**Fig. 4****: Single- and dual-blastomere haplotyping identifies the disease-carrying allele in the embryo.** **Fig. 4** (A-D) Haplotypes of the paternally inherited chromosome 22, SNPs within the microdeletion were excluded from the analysis (small dark grey bars). Alleles that trace back to the grandmaternal and grandpaternal chromosomes are indicated in light grey and dark grey respectively. Fig. 4 (A) Three single-blastomere derived haplotypes. Fig. 4 (B) Three dual-cell haplotypes derived from three different virtual genotypes respectively. The virtual genotypes consist of concordant SNP-calls in two single blastomeres. Fig. 4 (C) Smoothing result of the haplotypes shown in Fig. 4A. Fig. 4 (D) Smoothing result of the haplotypes shown in Fig. 4B.

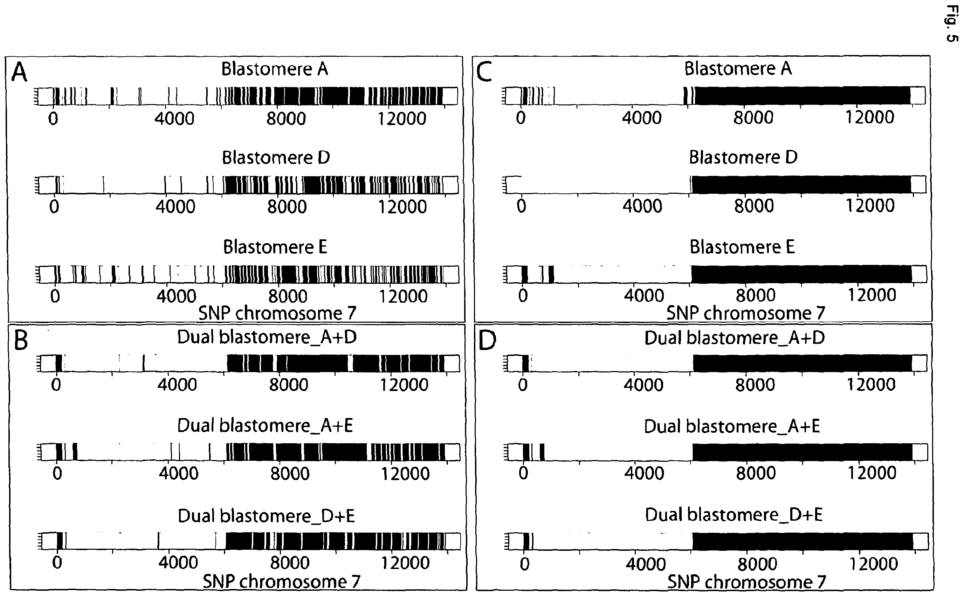

**Fig. 5****: Single- and dual-blastomere haplotyping allows pinpointing homologous recombination sites.** Fig. 5 (A-D) Haplotypes of the paternally inherited chromosome 7. Alleles that trace back to the grandmaternal and grandpaternal chromosomes are indicated in light grey and dark grey respectively. Fig. 5 (A) Three single-blastomere derived haplotypes. Fig. 5 (B) Three dual-cell haplotypes derived from three different virtual genotypes respectively. The virtual genotypes consist of concordant SNP-calls of two single blastomeres. Fig. 5 (C) Smoothing result of the haplotypes shown in Fig. 5A. Fig.5 (D) Smoothing result of the haplotypes shown in Fig. 5B.

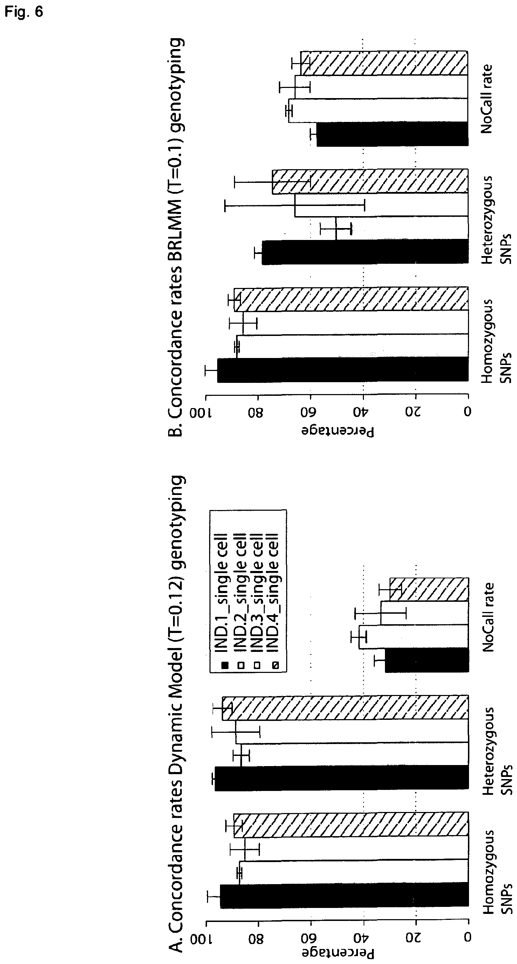

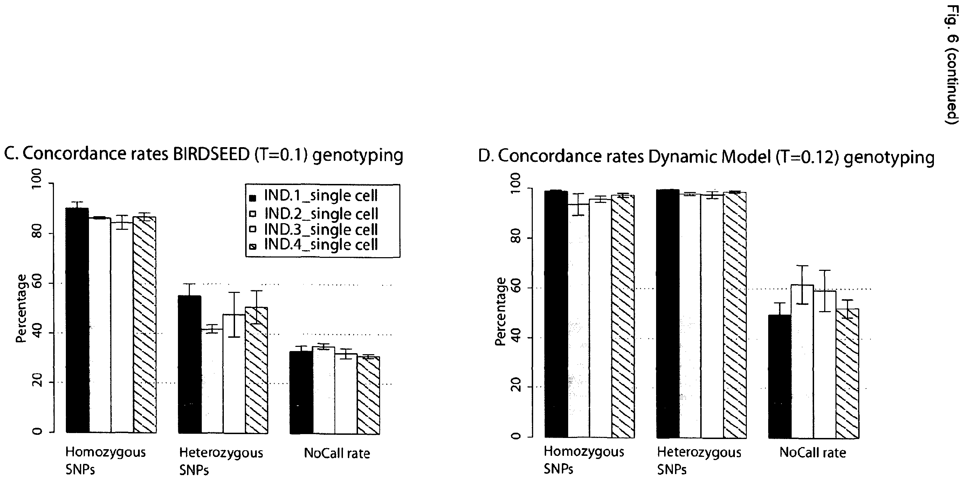

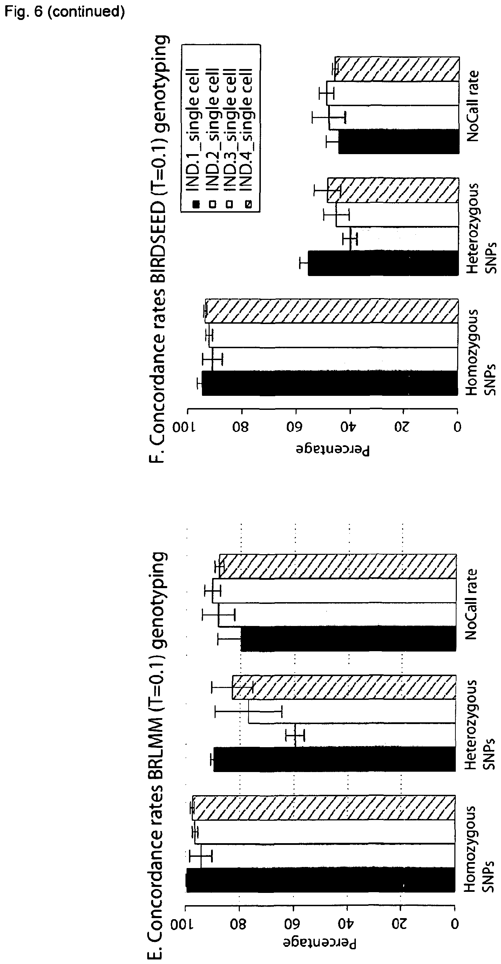

**Fig. 6****: Comparison of genotyping algorithms for single- and dual-cell SNP-typing accuracy.** (A-C) Each panel shows for four EBV-transformed lymphoblastoid cell lines the average concordance (± Standard Deviation %) of single-cell homozygous and heterozygous SNPs with the reference genotypes as well as the NoCall rate. The single cells (3 per cell line) were genotyped using either Dynamic Model at threshold 0.12 (A), BRLMM at threshold 0.1 (B) or BIRDSEED at threshold 0.1 (C). (D-F) Similar analysis for dual-cell derived genotypes that consist of SNP-calls concordant in two single-cell genotypes determined using either Dynamic Model at threshold 0.12 (D), BRLMM at threshold 0.1 (E) or BIRDSEED at threshold 0.1 (F). Three different dual-cell derived genotypes were evaluated per cell line.

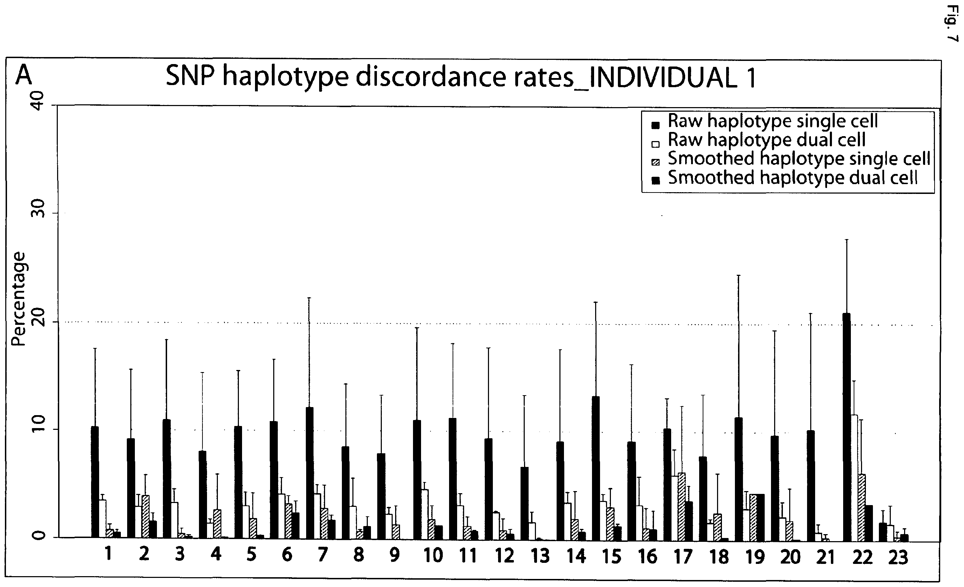

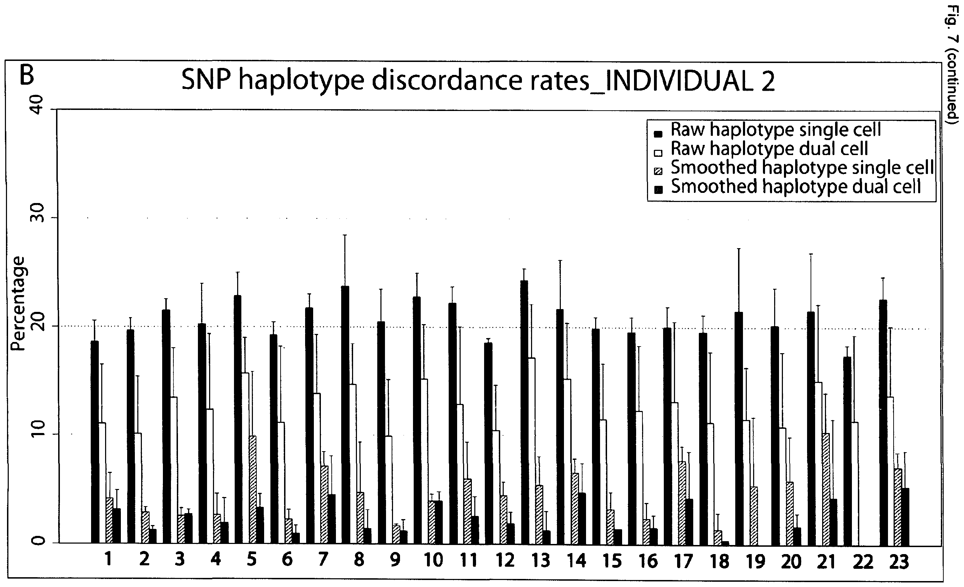

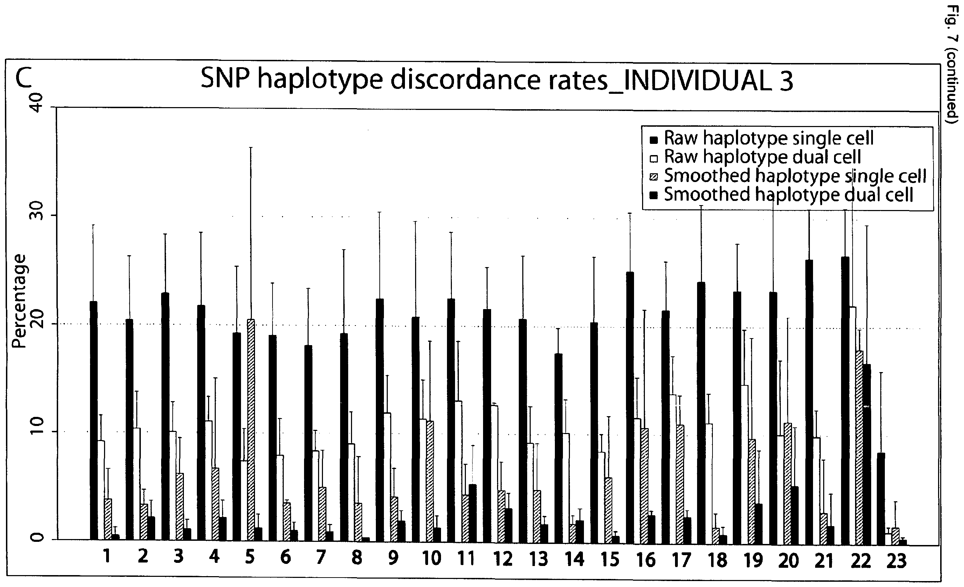

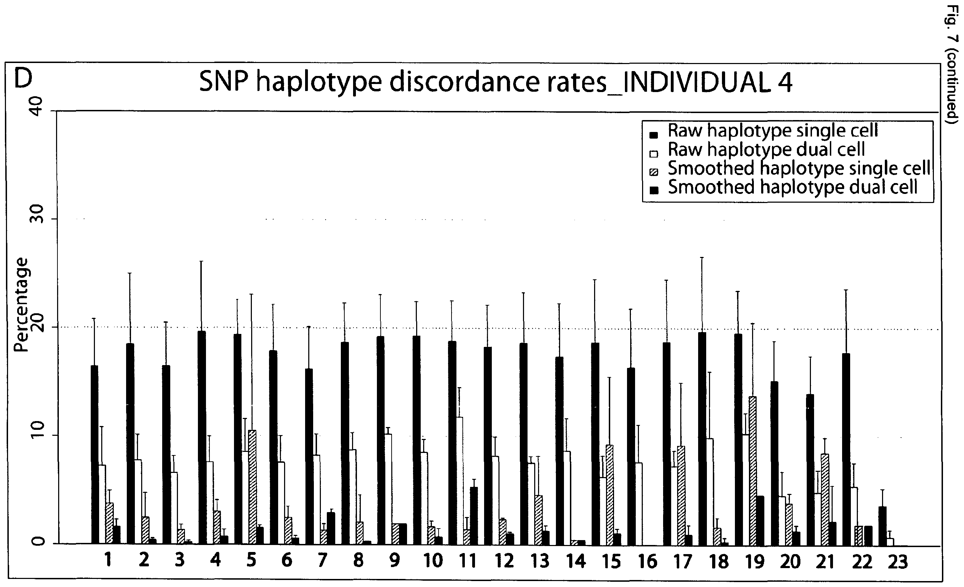

**Fig. 7****: SNP-haplotype discordance rates in four individuals.** Per individual the SNP haplotype discordance rates (± Standard Deviation %) for all maternally inherited chromosomes are shown using either single-cell (n=3) derived haplotypes, dual-cell (n=3) derived haplotypes, smoothed single-cell (n=3) derived haplotypes or smoothed dual-cell (n=3) derived haplotypes. (A-D) Results for individual 1-4 respectively.

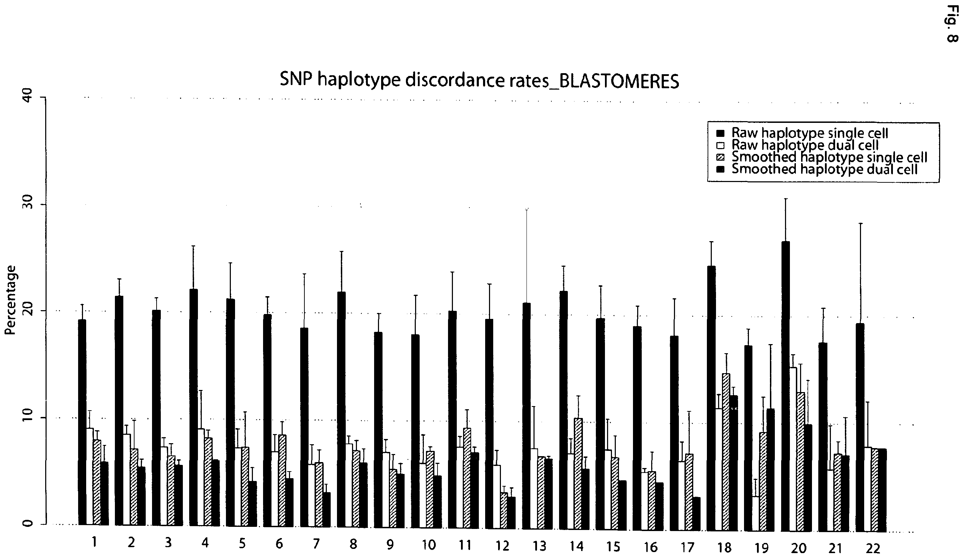

**Fig. 8****: SNP-haplotype discordance rates in blastomeres.** SNP haplotype discordance rates (± Standard Deviation %) for all paternally inherited autosomes are shown using either single-blastomere (n=3) derived haplotypes (black), dual-blastomere (n=3) derived haplotypes (white), smoothed single-blastomere (n=3) derived haplotypes (light grey) or smoothed dual-blastomere (n=3) derived haplotypes (dark grey). A reference haplotype was computed from the concordant SNP-calls among the three single-blastomere derived genotypes.
**Fig. 9****: Flowchart of the present invention.** The method of the present invention for single cell or dual-cell haplotyping comprises preferably the following steps: (1) Pick a single cell; (2) lyse the single cell; (3) amplify the single cell DNA e.g. by using multiple displacement amplification (MDA) technology; (4) massively parallel SNP-typing (=genotyping) of the amplified single cell DNA; (5) optional step: select only the heterozygous SNP-calls from the computed single-cell genotypes for subsequent haplotype reconstruction; (6) optional step: generate a virtual genotype consisting of SNP-calls concordant between two separately genotyped single cells; (7) reconstruct the haplotype of the single cell's genotype (= single cell haplotyping; with or without optional step 5, but without optional step 6) or virtual genotype (from optional step 6 = dual cell haplotyping): in this step the haplotyping can be performed using e.g. MERLIN software; (8) simplify interpretation of the generated single- and/or dual-cell 'raw' haplotypes obtained in the reconstruction by reassigning the allelic allocation of haplotype blocks smaller than a certain amount of SNPs (threshold) or by reassigning the allelic allocation of haplotype blocks using image processing techniques (e.g. a 1D median filter). Interpretation of single-cell and/or dual-cell haplotypes may be further simplified by integrating single-cell and/or dual-cell haplotypes that were computed by different methods and different smoothing methods and/or by integration with single-cell copy number profiles. 'Regular' haplotyping indicates haplotyping method starting from DNA that is extracted from multiple cells.
   Legend to figure 9: ^{a}Genome-wide SNP-typing method; ^{b}Genome-wide haplotype reconstruction method (using all SNPs of the single- or dual-cell genotype, or a selection thereof); ^{c}Smoothing algorithm(s) of the present invention: Removal of false-positive parental homologues recombination sites and erroneous allelic assignments; ^{d}Simplified interpretation enabled by smoothed haplotypes. Simplified interpretation could further include haplotype data integration of multiple single- or dual-cell haplotype computations and/or integration with single-cell copy number profiles.
**Fig. 10****: Flowchart of the smoothing algorithm.** A three-step smoothing algorithm for single-cell haplotypes was designed that re-adjusts the allelic allocation of small haplotype blocks to the grandparental assignments of the flanking large haplotype blocks. In a first cycle, this algorithm will remove potential false positive recombination sites between two flanking haplotype blocks larger than threshold 1 only if the latter are assigned to the same grandparent. Furthermore, the grandparental origin of the haplotype blocks smaller than the threshold 1 and located at the p- or q-terminus of a chromosome will be adjusted to the first haplotype block larger than threshold 1 if the p- or q-terminal haplotype blocks below the threshold 1 are collectively smaller than 500 SNPs. To refine the allelic assignments between haplotype blocks larger than 200 SNPs and allocated to a different grandparent, thresholds 2 and 3 were applied on the smoothed haplotypes in a second and third cycle of the algorithm respectively.

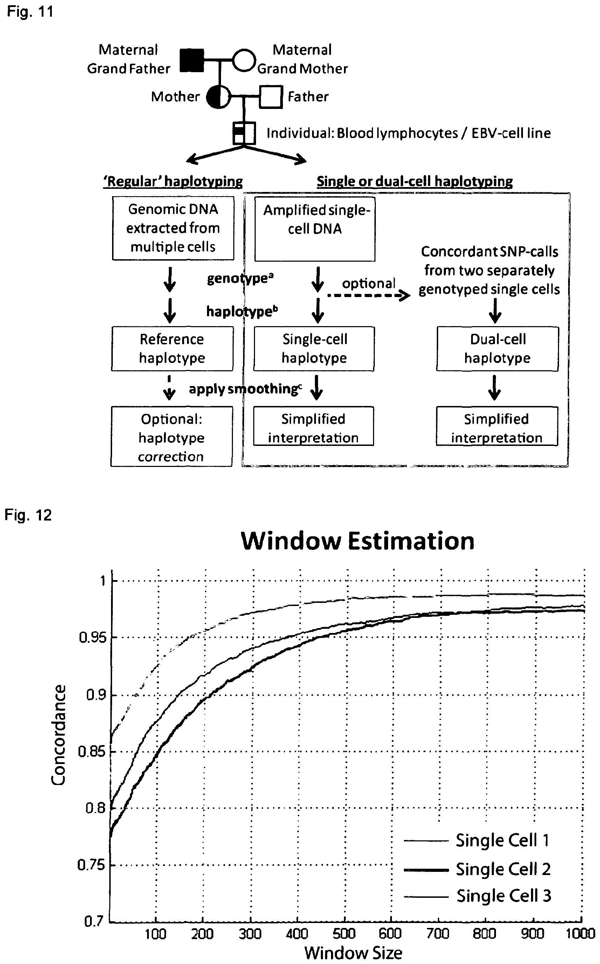

**Fig. 11****: Flowchart of Example 1.** Regular, single- and dual-cell haplotyping methods applied to the indicated sibling of the family.
   Legend to figure 11: ^{a}Genome-wide SNP-typing method; ^{b}Genome-wide haplotype reconstruction method; ^{c}Removal of false-positive parental homologous recombination sites and erroneous allelic assignments.
**Fig. 12****: Window optimization for the non-linear smoothing algorithm.** The Y-axis represents the single-cell haplotype concordance with the reference haplotype. The X-axis represents the applied non-linear smoothing window size. The results for three single cells (in different greyscales) of are shown.

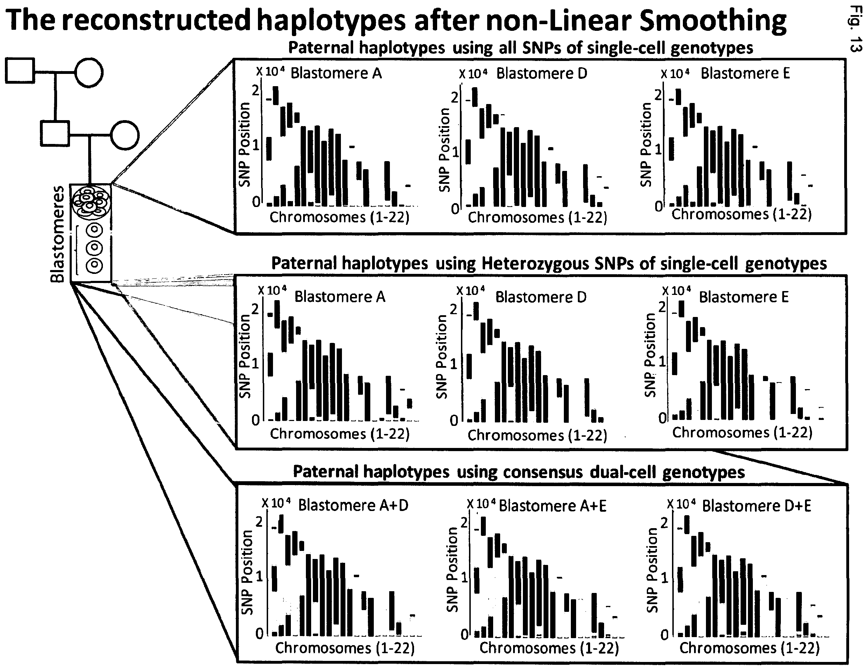

**Fig. 13****: Non-linear smoothed SNP-haplotypes of human blastomeres.** Genome-wide non-linear smoothed haplotypes (using the 1D median filter approach) resulting from three haplotyping Merlin input formats (being 1. raw single-cell genotype inputs, 2. inputs consisting of only heterozygous SNPs from raw single-cell genotypes, and 3. virtual dual-cell genotype inputs). The X-axis represents the 22 autosomes, and the Y-axis represents the relative SNP position for each chromosome. High concordances can be noticed between the haplotypes estimated for the different single cells as well as between the haplotypes resulting from the different Merlin input-genotype settings (either raw single-cell genotype inputs (top panel), heterozygous SNP inputs of the single-cell genotypes (middle panel), or virtual dual-cell genotype inputs (lower panel)).

### Definitions

- Genotype = any allelic constitution of a locus, chromosome or genome
- Haplotype = any sequence of syntenic alleles of a locus, chromosome or genome
- Single-cell haplotyping = any determination of the sequence of syntenic alleles from single-cell derived genotypes
- Dual-cell genotyping = determination of the concordant genetic polymorphism calls in two separately genotyped single-cells
- Dual-cell haplotyping = any determination of the sequence of syntenic alleles from concordant genetic polymorphism calls in two separately genotyped single-cells

### Detailed description of the present invention

The method of the present invention comprises preferably the following steps (see also the 'flow-chart of the present invention' in section 3):
1. Pick a single cell;
2. Lyse the single cell;
3. Amplify the single cell DNA e.g. using multiple displacement amplification (MDA) technology;
4. Massively parallel genetic polymorphism typing (=genotyping) of the amplified single cell DNA; in this step the massively parallel genetic polymorphism typing can e.g. be performed by hybridizing the amplified single cell DNA onto an Affymetrix high resolution SNP-array and subsequently using the Dynamic Model algorithm (Di et al. 2005) for interpreting Affymetrix GeneChip SNP-probe intensities;
5. Optional step: select only the heterozygous genetic polymorphism calls from the computed single-cell genotypes for subsequent haplotype reconstruction;
6. Optional step: generate a virtual genotype consisting of genetic polymorphism calls concordant between two separately genotyped single cells;
7. Reconstruct the haplotype of the single cell's genotype (= single-cell haplotyping; with or without optional step 5, but without optional step 6) or virtual genotype (from optional step 6 = dual cell haplotyping): in this step the haplotyping can be performed using e.g. MERLIN software;
8. Simplify interpretation of the generated single- and/or dual-cell 'raw' haplotypes using the designed smoothing algorithms.

The different steps are explained in more detail below:

### A. Single cell lysis, DNA amplification and purification

Step 1: Single cells are transferred to a 200 µl PCR tube containing 1.5 µl alkaline lysis buffer (ALB; 200 mM KOH and 50 mM DTT) by mouth controlled pipetting using 75 µm stripper tips (MidAtlantic Diagnostics, Mount Laurel, USA). An aliquot from the last washing droplet is taken as a negative control. Step 2: The samples are stored at -20°C for at least 30 min and are further incubated for 10 min at 65°C prior to the multiple displacement amplification (MDA) reaction. Step 3: Single cell (SC) DNA is amplified following an MDA-approach (Spits et al. 2006) with Genomi Phi V2 (GE Healthcare, Piscataway, NJ). The MDA products are purified and resuspended in 100 µl of elution buffer (High Pure PCR Product Purification Kit, Roche, Basel, Switzerland). All amplification products are quantified with a spectrophotometer (Nanodrop ND-1000 spectrophotometer; Nanodrop Technologies, Rockland, DE). SC-amplifications yielding less than 2 µg DNA are preferably not further used.

Alternatives include any single cell lysis, DNA amplification (DOP-PCR, PEP-PCR, ligation-mediated PCR, alu-PCR, PicoPlex whole genome amplification...) and DNA purification method.

### B. Single-cell genome-wide genetic polymorphism typing & dual-cell genotyping

Step 4: To enable genome-wide genetic polymorphism typing and subsequent haplotyping of single human cells, Affymetrix GeneChip Human Mapping 250K NspI arrays are performed using 250 ng of single cell amplified DNA according to manufacturer's instructions. The obtained SNP-probe intensities are subsequently interpreted using the Dynamic Model algorithm set at SNP-confidence threshold 0.12 (Di et al. 2005). Optional step 5: Only heterozygous SNPs from single cell genotypes are further applied. Optional step 6: Virtual genotypes consisting of the concordant SNP-calls from two separate single-cell analyses (= dual-cell derived genotypes) are created e.g. using Tinn-R 2.2.0.2 and R 2.8.0.

Alternatives include any genome-wide SNP-typing platform (including lower and higher resolution platforms from Affymetrix [e.g. GeneChip Human Mapping 250K Styl array, GeneChip Human Mapping 500K array, Genome-Wide Human SNP Array 5.0, Genome-Wide Human SNP Array 6.0, custom made arrays,...] and Illumina [e.g. HumanOmni1-Quad, Human1M-Duo, Human660W-Quad, HumanCytoSNP-12, custom made arrays,...]) with or without modifications to the manufacturer's instructions and subsequent genotyping software (RLMM, BRLMM, Birdseed, GenomeStudio Genotyping Module (Illumina),...). Alternatives also include any massively parallel sequencing approaches and the use of any type of polymorphic genetic variation for single- or dual-cell haplotyping.

### C. Single- and dual-cell haplotyping

Step 7: Genome-wide haplotype reconstruction of the single-cell or dual-cell derived genotype is performed with the MERLIN algorithm using the most likely pattern of gene flow option (**--best** command line option) (Abecasis et al. 2002). The '.ped', '.map' and '.dat' files are generated using Tinn-R 2.2.0.2 and R 2.8.0 or Matlab. As a requirement of MERLIN, every genetic polymorphism has to have a unique genetic distance. Sex-averaged SNP genetic distances extrapolated from the deCODE map (Kong et al. 2002) are used and are listed in the NetAffx annotation file version NA27 (11/25/08).

Alternatives include any haplotyping software (Stephens and Donnelly 2003; Gao et al. 2009; Handyside et al. 2009) and usage of any sex-specific/averaged genetic maps (deCODE map (Kong et al. 2002), the Marshfield map (Broman et al. 1998), the SLM1 map generated from unpublished data from Affymetrix and Dr. Aravinda Chakravarti group at Johns Hopkins University. It was based on genotypes for 2,022 microsatellite markers and 6,205 SNPs. ...) or usage of known haplotypes and haplotype frequencies in a given ethnic background (Frazer et al. 2007).

### D. Smoothing of single and dual-cell raw haplotypes

Genome-wide haplotyping of diploid single cells has been precluded mainly due to genotype errors introduced by single cell DNA-amplification artifacts like random allele-dropout (ADO) and preferential amplification (PA) (step 3) as well as by false algorithmic interpretations of genetic polymorphism detection (besides ADO and PA phenomena, allele-drop in (ADI) is observed as well) (step 4). The most problematic genetic polymorphism typing errors for haplotyping (step 7) are those that do not lead to Mendelian inconsistencies thereby introducing false allelic assignments and false homologous recombination sites. Hence, algorithm(s) are required to reliably interpret raw single-cell haplotype interpretation (step 8), even if one can circumvent issues with allele-dropout and preferential amplification by using only the single-cell derived heterozygous genetic polymorphisms.

Step 8: The smoothing algorithm facilitates the interpretation of the generated single and/or dual cell 'raw' haplotypes by adjusting wrong allelic allocations of haplotype blocks. Considering the ease of excluding or including genetic polymorphisms in genome-wide scans and downstream analyses, the existence of laboratory-, user- and algorithmic-bias in genome-wide genetic polymorphism typing, the below defined thresholds should not be considered absolute, but flexible and amendable to the genetic polymorphism typing platform/algorithm used, the genetic polymorphism annotation file used, the laboratory, the user etc.

To facilitate the interpretation of the raw single- or dual-cell derived haplotypes and to pinpoint genetic crossovers more accurately from the raw haplotypes, apply a three-step smoothing algorithm (see Fig. 10) for single- and dual-cell haplotypes. The smoothing algorithm of the present invention adjusts the allelic allocation of haplotype blocks smaller than threshold 1 - in a preferred embodiment of the present invention, the threshold 1 for haplotype block size is set to 200 SNPs - to the grandparental assignments of the flanking haplotype blocks larger than threshold 1. This algorithmic design is based on the finding that raw haplotype blocks larger than threshold 1 are mostly assigned to the correct allele. In a first step of the algorithm, the user-tunable threshold 1 for haplotype block size is set (see Fig.10 STEP1). As such, this algorithm will remove potential false positive recombination sites between two flanking haplotype blocks larger than threshold 1 only if the latter are assigned to the same grandparent. Furthermore, the grandparental origin of the haplotype blocks smaller than threshold 1 and located at the p- or q-terminus of a chromosome will be adjusted to the first haplotype block larger than threshold 1 if the p- or q-terminal haplotype blocks below threshold 1 are collectively smaller than 500 SNPs (the latter is user-tunable as well and can be arbitrarily defined). To refine the allelic assignments between haplotype blocks larger than threshold 1 and allocated to a different grandparent (see Fig. 10 STEP 2 & 3), thresholds 2 and 3 - in a preferred embodiment of the present invention, thresholds 2 and 3 for haplotype block size are set to 50 and 200 SNPs respectively - are applied on the smoothed haplotypes (after STEP1) in a second and third step of the algorithm respectively.

The algorithm for smoothing of the single-cell haplotypes was designed in Tinn-R 2.2.0.2 and R 2.8.0.

In an alternative approach of step 8: A non-linear smoothing algorithm facilitates the interpretation of the generated single and/or dual cell 'raw' haplotypes as well as the identification of genetic crossovers from the raw haplotypes by adjusting allelic allocations of one or more genetic polymorphisms and/or intermediate regions. In a preferred embodiment of the present invention a "1 D median filter" (Pratt 1978) is applied for this non-linear smoothing algorithm. A median filter is an image processing technique to reduce noise, but can preserve edges while removing noise. In this non-linear smoothing algorithm, the "1 D median filter" uses a sliding window (expressed in number of genetic polymorphisms) and the adjustment of allelic assignments (expressed in binary format) within each window. The adjustment is performed according to the median value of the window. The size of the window is defined by the user. In a preferred embodiment of the current algorithm we used a window of 450 SNPs for chromosome 1 (see Fig. 12). The non-linear smoothing algorithm then applies a window that is based on the number of genetic polymorphisms in each chromosome (in a preferred embodiment of the non-linear smoothing algorithm: the chromosome-specific window = (number of genetic polymorphisms in a specific chromosome) / (number of genetic polymorphisms in chromosome 1) x window for chromosome 1). Finally, the non-linear smoothing algorithm compares the smoothed haplotype blocks with the raw haplotype blocks and determines whether the majority of the genetic polymorphisms in the raw haplotype blocks are assigned to the same allele as in the smoothed haplotype block. If this was not the case, the raw haplotype was maintained. The algorithm was designed in Matlab.

Furthermore, additional rules can be included. In a preferred embodiment single-cell haplotypes that are computed and smoothed by different approaches are integrated for haplotype concordance simplifying further the interpretation. In a preferred embodiment of the present invention: single-cell haplotypes are also integrated with single-cell DNA copy number profiles determined by existing methods (Vanneste et al. 2009) for reliable single-cell genotype and haplotype interpretation. Furthermore additional rules in the smoothing and interpretation steps that make use of haplotype block size (e.g. defining genetic polymorphism haplotype block size thresholds for adjusting or keeping the allelic assignment of haplotype blocks based on the meiotic crossover interference principle, the calculation of a ratio of the number of genetic polymorphisms assigned to different grandparental origin and adjustment of the allelic assignments only of this ratio meets user-defined criteria,...) could be included in the methods decribed above as well. Also additional steps that make use of known haplotypes and haplotype frequencies in the different ethnic backgrounds could be added (Frazer et al. 2007). Finally, the smoothing algorithm described above could be equipped with information of known (hot)spots of homologous recombination (Frazer et al. 2007).

The smoothing algorithms of the present invention are particularly useful to facilitate interpretation of haplotypes that are based on the results of single nucleotide polymorphism (SNP)-arrays or massively parallel DNA-sequencers. A skilled person in the art will appreciate the applicative value of the aforementioned methods for reliable haplotype estimation of a (single-cell) genome or locus when any genotyping method is performed on whole-genome amplified (single-cell) DNA and is followed by any haplotyping method.

The methods described in this application for single- and/or dual-cell haplotyping with smoothing can be used/applied to human and animal cells for embryo selection purposes, for genetic studies of heterogeneous tissues consisting of cells with different allelic constitutions, or for forensic research

### 5. Examples

### Example 1: Haplotyping of EBV-transformed human lymphoblastoid cells

### Genotyping of single cells

The method was established using four EBV-transformed lymphoblastoid cell lines derived from four different individuals respectively. Genomic DNA isolated from multiple blood cells of these individuals as well as MDA-DNA from three single cells per cell line were hybridized to Affymetrix 250K SNP-arrays. The obtained SNP-probe intensities were interpreted by three different algorithms - Dynamic Model (Di et al. 2005), BRLMM and Birdseed - that are optimized for typing SNPs in diploid genomes not subjected to MDA, allele drop-out (ADO) or preferential amplification (PA).

SNP-typing accuracy of the MDA single-cell DNA was determined by aligning the single-cell genotypes with the reference genotypes obtained from the corresponding non-amplified genomic DNA. Although the different algorithms had minor effects on the concordances of the single-cell homozygous SNPs with the reference SNPs, a significant effect was noted on the concordances of the heterozygous SNPs (Table 1 and Fig. 6). The Dynamic Model algorithm outperforms both the BRLMM and Birdseed algorithms for typing heterozygous SNPs in single human cells. Using a SNP-typing confidence threshold of 0.12 in the Dynamic Model algorithm on average 89.04 % (± 5.0 % SD) of the homozygous SNPs and 91.24 % (± 6.0 % SD) of the heterozygous SNPs in the single cells had a match with the corresponding genotype determined with genomic DNA isolated from multiple cells.

Furthermore, of the SNPs that are heterozygous in the non-amplified genomic DNA samples on average 29.38 % (±11.30 % SD), 12.41 % (±4.81 % SD) and 32.91 % (±6.33 % SD) are homozygous in the corresponding single cells when genotyped with the Dynamic Model (Di et al. 2005), BRLMM or Birdseed algorithm respectively. Part of these inconsistencies may be attributed to whole genome amplification artifacts, like allele drop out or preferential amplification of particular alleles of a single cell's genome (Spits et al. 2006; Handyside et al. 2009). However, it is clear that different statistical algorithms introduce different calling errors.

Single-cell DNA-fingerprints computed by the Dynamic Model were further explored for haplotype reconstruction.

**Table 1: Comparison of genotyping algorithms for single-cell SNP-typing accuracy.**

| **Concordance** | **DM ^{c}** | **BRLMM ^{c}** | **BIRDSEED ^{c}** |
|---|---|---|---|
| SC-hmz SNPS ^{a} | 89.04 (± 5) | 89.50 (± 4.93) | 86.97 (± 2.77) |
| SC-htz SNPs ^{b} | 91.24 (± 6) | 67.26 (± 17.29) | 48.91 (± 7.32) |

| | | | |
|---|---|---|---|
| ^{a} Concordance of single-cell homozygous SNPs with the reference genotype ^{b} Concordance of single-cell heterozygous SNPs with the reference genotype ^{c} Data are represented as mean % (± standard deviation %) Reference genotypes are Dynamic Model-typed genomic DNA samples (threshold 0.12) | | | |

### Single- and dual-cell haplotyping

To investigate the possibility for reconstructing haplotypes from single-cell derived genotypes, we first determined for four individuals belonging to three different families, a reference allelic sequence of the maternally inherited autosomes and sex chromosome (Figure 11). To this end, genomic DNA of the four individuals as well as of their parents and maternal grandparents were typed for 250K SNPs and the haplotypes were computed using the MERLIN algorithm (Abecasis et al. 2002). Two recombination sites each in a different individual were validated by STR-marker analysis (data not shown). Subsequently, single-cell derived haplotypes were determined by swapping the genotypes of the four individuals with single-cell derived genotypes in the MERLIN-pedigree files. Three single lymphoblastoid cells were phased per individual.

The resulting single-cell derived haplotypes contained significantly more haplotype blocks consisting of fewer consecutive SNPs when compared to the reference haplotypes (Fig.1A). As a result of these false-positive recombination sites the relative amount of autosomal SNPs in haplotype discordance is approximately 17.72 % (± 5.0 % SD) in the computed single-cell haplotypes (Fig.1E, Fig. 7), which makes it difficult to discern the exact positions of maternal genetic crossovers between grandparental homologous chromosomes on the basis of single-cell derived haplotypes.

The discrepancies between the single-cell derived haplotypes and the reference allelic sequences are due to the genotyping errors of the single-cell MDA-DNA. To derive more reliable genotypes, an algorithm was designed that creates virtual genotypes consisting of SNP-calls that are concordant in two single-cell genotypes of the same individual. On average 96.32 % (± 2.88 % SD) of the homozygous SNPs and 98.46 % (± 1.11 % SD) of the heterozygous SNPs in this dual-cell analysis had a match with the reference genotype (Fig.6). Reconstructing haplotypes with these virtual genotypes significantly lowered the number of haplotype blocks, false-positive recombination sites and haplotype discordance of the autosomal SNPs (8.77 % ± 4.24 % SD) (Fig.1B, 1E, Fig. 7), allowing to visually distinguish the true allelic sequence in synteny.

### Smoothing of haplotypes

Means to further simplify the analysis of single- as well as dual-cell derived sequences of syntenic alleles were developed. Phasing genotypes of single cells frequently generates numerous small haplotype blocks that do not match the reference haplotype. To evaluate the potential for generating an algorithm that rectifies at least part of these discrepant allelic assignments, histograms were created for the sizes of the haplotype blocks that are respectively concordant, discordant and spanning homologous recombination sites when compared to the reference haplotype (Fig.2A). This revealed that the majority of haplotype blocks without a reference match are smaller than 200 consecutive SNPs (Fig. 2A-C). Based on this observation, a three-step smoothing algorithm for single-cell haplotypes was designed that re-adjusts the allelic allocation of small haplotype blocks to the grandparental assignments of the flanking large haplotype blocks. In a first cycle of the algorithm, the user-tunable threshold for haplotype block size was set to 200 SNPs. As such, this algorithm will remove potential false positive recombination sites between two flanking haplotype blocks larger than 200 SNPs only if the latter are assigned to the same grandparent. Furthermore, the grandparental origin of the haplotype blocks smaller than the threshold and located at the p- or q-terminus of a chromosome will be adjusted to the first haplotype block at least 200 SNPs wide if the p- or q-terminal haplotype blocks below the threshold are collectively smaller than 500 SNPs. To refine the allelic assignments between haplotype blocks larger than 200 SNPs and allocated to a different grandparent, thresholds of 50 and 200 SNPs were applied on the smoothed haplotypes in a second and third cycle of the algorithm respectively.

Haplotypes derived from the single- and the dual-cell genotypes were smoothed with this algorithm. Smoothing the syntenic grandparental allelic sequence derived from single-cell genotypes produced less SNPs in haplotype discordance (4.45 % ± 3.85 % SD) (Fig.1C,E, Fig. 7) when compared to haplotypes derived from SNP-calls concordant between two single-cell genotypes. Smoothing of the latter dual-cell derived haplotypes produced the best results (SNPs in haplotype discordance: 1.86 % ± 2.13 % SD) (Fig.1 D-E, Fig. 7).

To determine the accuracy of genetic crossover mapping using the smoothed single- and dual-cell haplotyping data, we aligned 175 homologous recombination sites that were detected in the four individuals. Computation of the frequency of haplotype concordance for 1000 SNPs surrounding these aligned genetic crossovers (Fig. 3A) with the reference allelic sequence, indicated that smoothed dual-cell derived haplotypes pinpoint crossovers more accurately than smoothed single-cell derived haplotypes. Indeed, for smoothed single- and dual-cell derived haplotypes a distance of approximately 250 and 50 SNPs flanking a genetic crossover respectively has to be considered to reach a frequency of >90 % of correct SNP-haplotype inference. The frequency of crossover-flanking SNPs assigned to the correct grandparental origin was higher for the smoothed than the 'raw' single-or dual-cell derived haplotypes (Fig. 3A-B).

In conclusion, this algorithm further facilitates pinpointing homologous recombination sites between grandparental chromosomes on inherited chromatids on the basis of single- or dual-cell analyses.

A second 'non-linear' smoothing algorithm was developed as well. This algorithm is based on a "1 D median filter". In general, a median filter is an image processing technique to reduce noise, but can preserve edges while removing noise. In this non-linear smoothing algorithm, the "1 D median filter" uses a sliding window (expressed in number of SNPs) and the adjustment of allelic assignments (expressed in binary format) within each window. The adjustment is performed according to the median value of the window of which the size is defined by the user. In a preferred embodiment of the current algorithm we used a window of 450 SNPs for chromosome 1 (see Fig. 12). We furthermore observed that there was a correlation between the density of SNP-markers in each chromosome and the optimal window size for raw haplotype interpretation of each chromosome. The non-linear smoothing algorithm, therefore, computes and applies a window that is based on the number of SNPs in each chromosome (the chromosome-specific window = (number of SNPs in a specific chromosome) / (number of SNPs in chromosome 1) x window for chromosome 1). Finally, this algorithm compares the smoothed haplotype blocks with the raw haplotype blocks and determines whether the majority of the SNPs in the raw haplotype blocks are assigned to the same allele as in the smoothed haplotype block. If this was not the case, the raw haplotype was maintained. The non-linear smoothing algorithm yielded superior haplotype discordance rates (3.40 % ± 2.05 % SD for EBV-lymphoblastoid cells of one individual and 1.65 % ± 0.51 % SD for EBV-lymphoblastoid cells of another individual).

### Example 2: Haplotyping of human blastomeres

To test whether this single- and dual-cell approach for haplotyping lymphoblastoid cells also works on human blastomeres, the grandparental allelic sequences on the paternally inherited autosomes in three single blastomeres from the same embryo were determined. This male embryo resulted from assisted reproductive technology applied for preimplantation genetic diagnosis (PGD) because the couple wished to circumvent the paternal transmission of a 22q11 deletion to their child. FISH-analysis on two additional blastomeres revealed that this embryo carried the 22q11 deletion (data not shown). We were able to confirm this FISH-result by reconstructing the haplotypes of the paternally inherited chromosomes in the three blastomeres.

By probe intensity-cluster graph analysis of SNPs located within the 22q11 deletion it was proven that the deletion occurred *de novo* on a grandmaternal allele in the father (data not shown). Haplotype reconstruction of the Dynamic Model-derived genotypes of the three single blastomeres and of three corresponding virtual genotypes resulting from concordant SNP calls in two single blastomeres revealed that the 22q11 locus was embedded in a grandmaternal haplotype block in these blastomeres (Fig.4A-B). This analysis was performed by including and excluding (Fig.4A-B) the SNPs within the known 22q11 deletion region. Since SNPs within the deletion cannot be accurately typed false recombination sites were detected, this was avoided by excluding these SNPs from the haplotype reconstruction. After smoothing of the computed haplotypes, no homologous recombination sites were found on this paternally inherited chromatid of chromosome 22 (Fig.4C-D). Similar results were obtained for eight other autosomes, while the remaining inherited autosomes showed sites of homologous recombination between grandparental chromosomes (Fig.5A-D).

Furthermore, a reference haplotype of this embryo was computed from the concordant SNP calls in the three single blastomeres. Approximately 20.3 % (± 3.4 % SD) of the autosomal SNPs in haplotypes derived from single blastomeres were found to be discordant with this reference allelic sequence (Fig. 8). Blending the genotypes of these blastomeres two by two lowered the haplotype discordance rate of the autosomal SNPs to 7.54 % (± 1.90 % SD), while smoothing of the single- and dual-cell derived haplotypes yielded discordance rates of 7.84 % (± 1.60 % SD) and 6.08 % (± 1.20 % SD) respectively. Although a reference haplotype derived from non-amplified genomic DNA extracted from multiple cells is lacking for this embryo, these results are comparable to the haplotype reconstructions of the single EBV-transformed lymphoblastoid cells.

Application of the non-linear smoothing algorithm to haplotypes resulting from different single-cell genotype Merlin input-format strategies (being 1. raw single-cell genotype inputs, 2. inputs consisting of only heterozygous SNPs from raw single-cell genotypes, and 3. virtual dual-cell genotype inputs) further demonstrated high genome-wide haplotype concordance and enabled the genome-wide detection of the meiotic recombination events between the paternal homologous chromosomes as well (fig.13).

In conclusion, our results prove the principle that single- and dual-blastomere haplotyping in combination with haplotype smoothing allow to assess allele-specific inheritance of loci in embryos.

The methods described in this application for single- and/or dual-cell haplotyping with or without smoothing can be used/applied to human and animal cells for embryo selection purposes, for genetic studies of heterogeneous tissues consisting of cells with different allelic constitutions, or for forensic research.

### References

1. Abecasis GR, Cherny SS, Cookson WO, Cardon LR. 2002. Merlin--rapid analysis of dense genetic maps using sparse gene flow trees. Nat.Genet. 30:97-101
2. Altshuler D, Daly MJ, Lander ES. 2008. Genetic mapping in human disease. Science 322:881-888
3. Broman KW, Murray JC, Sheffield VC, White RL, Weber JL. 1998. Comprehensive human genetic maps: individual and sex-specific variation in recombination. Am.J.Hum.Genet. 63:861-869
4. Di X, Matsuzaki H, Webster TA, Hubbell E, Liu G, Dong S, Bartell D, Huang J, Chiles R, Yang G, et al. 2005. Dynamic model based algorithms for screening and genotyping over 100 K SNPs on oligonucleotide microarrays. Bioinformatics. 21:1958-1963
5. Frazer KA, Ballinger DG, Cox DR, Hinds DA, Stuve LL, Gibbs RA, Belmont JW, Boudreau A, Hardenbol P, Leal SM, et al. 2007. A second generation human haplotype map of over 3.1 million SNPs. Nature 449:851-861
6. Frazer KA, Murray SS, Schork NJ, Topol EJ. 2009. Human genetic variation and its contribution to complex traits. Nat.Rev.Genet. 10:241-251
7. Gao G, Allison DB, Hoeschele I. 2009. Haplotyping methods for pedigrees. Hum.Hered. 67:248-266
8. Goddard ME and Hayes BJ. 2009. Mapping genes for complex traits in domestic animals and their use in breeding programmes. Nat.Rev.Genet. 10:381-391
9. Handyside AH, Harton GL, Mariani B, Thornhill AR, Affara NA, Shaw MA, Griffin DK. 2009. Karyomapping: a Universal Method for Genome Wide Analysis of Genetic Disease based on Mapping Crossovers between Parental Haplotypes. J.Med.Genet. 47:651-658.
10. Iwamoto K, Bundo M, Ueda J, Nakano Y, Ukai W, Hashimoto E, Saito T, Kato T. 2007. Detection of chromosomal structural alterations in single cells by SNP arrays: a systematic survey of amplification bias and optimized workflow. PLoS.ONE. 2:e1306
11. Johnson DS, Gemelos G, Baner J, Ryan A, Cinnioglu C, Banjevic M, Ross R, Alper M, Barrett B, Frederick J, et al. 2010. Preclinical validation of a microarray method for full molecular karyotyping of blastomeres in a 24-h protocol. Hum.Reprod. 25:1066-1075
12. Kong A, Gudbjartsson DF, Sainz J, Jonsdottir GM, Gudjonsson SA, Richardsson B, Sigurdardottir S, Barnard J, Hallbeck B, Masson G, et al. 2002. A high-resolution recombination map of the human genome. Nat.Genet. 31:241-247
13. Meuwissen TH, Hayes BJ, Goddard ME. 2001. Prediction of total genetic value using genome-wide dense marker maps. Genetics 157:1819-1829
14. Pratt, W.K. 1978. Digital Image Processing. In , pp. 330-333. John Wiley & Sons.
15. Renwick PJ, Trussler J, Ostad-Saffari E, Fassihi H, Black C, Braude P, Ogilvie CM, Abbs S. 2006. Proof of principle and first cases using preimplantation genetic haplotyping--a paradigm shift for embryo diagnosis. Reprod.Biomed.Online. 13:110-119
16. Spits C, Le Caignec C, De Rycke M, Van Haute L, Van Steirteghem A, Liebaers I, Sermon K. 2006. Whole-genome multiple displacement amplification from single cells. Nat.Protoc. 1:1965-1970
17. Stephens M and Donnelly P. 2003. A comparison of bayesian methods for haplotype reconstruction from population genotype data. Am.J.Hum.Genet. 73:1162-1169
18. Vanneste E, Voet T, Le Caignec C, Ampe M, Konings P, Melotte C, Debrock S, Amyere M, Vikkula M, Schuit F, et al. 2009. Chromosome instability is common in human cleavage-stage embryos. Nat.Med. 15:577-583

## Claims

1. A method for single cell haplotyping comprising the steps of
a. Picking a single cell;
b. Lysing said single cell;
c. Amplifying said single cell DNA;
d. Massively parallel genetic polymorphism typing of said amplified single cell DNA;
e. Reconstructing the haplotype of the single cell's genotype using haplotyping software using all genetic polymorphisms or a selection thereof;
f. Reassigning the allelic allocation of one or more genetic polymorphisms and/or intermediate regions, based on the information of neighboring genetic polymorphisms.

2. A method for dual cell haplotyping comprising the steps of
a. Picking two cells;
b. Lysing said two cells;
c. For each cell, amplifying the single cell DNA;
d. For each cell, massively parallel genetic polymorphism typing of the amplified single cell DNA;
e. Generating a virtual genotype consisting of genetic polymorphism calls concordant between the two separately genotyped single cells;
f. Reconstructing the haplotype of said virtual genotype using haplotyping software;
g. Reassigning the allelic allocation of one or more genetic polymorphisms and/or intermediate regions, based on the information of neighboring genetic polymorphisms.

3. The method of claim 1 or claim 2 wherein the reassigning the allelic allocation of one or more genetic polymorphisms and or intermediate regions is performed by reassigning the allelic allocation of haplotype blocks smaller than a certain amount of genetic polymorphisms.

4. The method of claim 3 wherein the reassigning the allelic allocation of haplotype blocks smaller than a certain amount of genetic polymorphisms makes use of the grandparental origin of haplotype blocks larger than said amount of genetic polymorphisms.

5. The method of any one of claims 3 to 4 wherein the reassigning the allelic allocation of haplotype blocks smaller than a certain amount of genetic polymorphisms is done by making use of the grandparental origin of the first left and right flanking haplotype blocks which are larger than the threshold.

6. The method of any one of claims 3 to 5 wherein haplotype blocks which are smaller, in amount of genetic polymorphisms, than a certain threshold T₁ are assigned to the first haplotype block on the left or right side of the haplotype block larger than the threshold T₁ only if these two haplotype blocks are assigned to the same grandparent.

7. A method for improving single cell or dual cell derived computed haplotypes comprising reassigning the allelic allocation of one or more genetic polymorphisms and/or intermediate regions based on the information of neighboring genetic polymorphisms.

8. The method of claim 7, wherein the reassigning the allelic allocation of one or more of the genetic polymorphisms and/or intermediate regions comprises reassigning the allelic allocation of haplotype blocks smaller than a certain amount of genetic polymorphisms.

9. The method of claim 8 wherein the reassigning the allelic allocation of haplotype blocks smaller than a certain amount of genetic polymorphisms makes use of the grandparental origin of haplotype blocks larger than said amount of genetic polymorphisms.

10. The method of any one of claims 8 or 9 wherein the reassigning the allelic allocation of haplotype blocks smaller than a certain amount of genetic polymorphisms is done by making use of the grandparental origin of the first left and right flanking haplotype blocks which are larger than the threshold.

11. The method of any one of claims 8 to 10 wherein haplotype blocks which are smaller, in amount of genetic polymorphisms, than a certain threshold T₁ are assigned to the first haplotype block on the left or right side of the haplotype block larger than the threshold T₁ only if these two haplotype blocks are assigned to the same grandparent.

12. The method of claim 1 or 2 wherein the reassigning the allelic allocation of one or more genetic polymorphisms and/or intermediate regions is based on image processing techniques.

13. The method of claim 12 wherein the image processing technique is the application of a "1 D median filter".

14. The method of any of the previous claims wherein the genetic polymorphism typing is performed by single nucleotide polymorphism typing.

15. The method of any of the previous claims wherein the genetic polymorphism typing is performed by DNA-sequencing.

16. The method of any of the previous claims wherein the single cell is a human or animal blastomere.

## Patentansprüche

1. Eine Methode der Einzelzell-Haplotypisierung, die folgende Schritte umfasst:
a. Auswählen einer Einzelzelle;
b. Lysieren der genannten Einzelzelle;
c. Amplifizieren der DNA der genannten Einzelzelle;
d. Massiv parallele genetische Polymorphismus-Typisierung der genannten amplifizierten Einzelzell-DNA;
e. Rekonstruieren des Haplotyps des Einzelzell-Genotyps mittels Haplotypisierungs-Software unter Verwendung aller genetischen Polymorphismen oder einer Auswahl davon;
f. Neuzuordnen der Allel-Allokation eines oder mehrerer genetischer Polymorphismen und/oder Zwischenregionen, basierend auf der Information von benachbarten genetischen Polymorphismen.

2. Eine Methode der Zweizell-Haplotypisierung, die folgende Schritte umfasst:
a. Auswählen von zwei Zellen;
b. Lysieren der genannten zwei Zellen;
c. Amplifizieren der Einzelzell-DNA jeder Zelle;
d. Massiv parallele genetische Polymorphismus-Typisierung der amplifizierten Einzelzell-DNA jeder Zelle;
e. Erzeugen eines virtuellen Genotyps, bestehend aus genetischen Polymorphismus-Calls, die zwischen den zwei separat genotypisierten Einzelzellen konkordant sind;
f. Rekonstruieren des Haplotyps des genannten virtuellen Genotyps mittels Haplotypisierungs-Software;
g. Neuzuordnen der Allel-Allokation eines oder mehrerer genetischer Polymorphismen und/oder Zwischenregionen, basierend auf der Information von benachbarten genetischen Polymorphismen.

3. Die Methode gemäß Anspruch 1 oder Anspruch 2, wobei das Neuzuordnen der Allel-Allokation eines oder mehrerer genetischer Polymorphismen und/oder Zwischenregionen durch Neuzuordnen der Allel-Allokation von Haplotyp-Blöcken erfolgt, die kleiner als eine bestimmte Menge an genetischen Polymorphismen sind.

4. Die Methode gemäß Anspruch 3, wobei zum Neuzuordnen der Allel-Allokation von Haplotyp-Blöcken, die kleiner als eine bestimmte Menge an genetischen Polymorphismen sind, der großelterliche Ursprung von Haplotyp-Blöcken, die größer als die genannte Menge an genetischen Polymorphismen sind, genutzt wird.

5. Die Methode gemäß einem der Ansprüche 3 bis 4, wobei das Neuzuordnen der Allel-Allokation von Haplotyp-Blöcken, die kleiner als eine bestimmte Menge an genetischen Polymorphismen sind, dadurch erfolgt, dass der großelterliche Ursprung der ersten linken und rechten flankierenden Haplotyp-Blöcke, die größer als der Schwellenwert sind, genutzt wird.

6. Die Methode gemäß einem der Ansprüche 3 bis 5, wobei Haplotyp-Blöcke, die in ihrer Menge an genetischen Polymorphismen kleiner als ein bestimmter Schwellenwert T₁ sind, dem ersten Haplotyp-Block auf der linken oder rechten Seite des Haplotyp-Blocks, der größer als der Schwellenwert T₁ ist, zugeordnet werden, nur wenn diese zwei Haplotyp-Blöcke demselben Großelternteil zugeordnet werden.

7. Eine Methode zur Verbesserung von aus Einzelzellen oder zwei Zellen abgeleiteten berechneten Haplotypen, die das Neuzuordnen der Allel-Allokation eines oder mehrerer genetischer Polymorphismen und/oder Zwischenregionen, basierend auf der Information von benachbarten genetischen Polymorphismen, umfasst.

8. Die Methode gemäß Anspruch 7, wobei das Neuzuordnen der Allel-Allokation eines oder mehrerer genetischer Polymorphismen und/oder Zwischenregionen das Neuzuordnen der Allel-Allokation von Haplotyp-Blöcken umfasst, die kleiner als eine bestimmte Menge an genetischen Polymorphismen sind.

9. Die Methode gemäß Anspruch 8, wobei zum Neuzuordnen der Allel-Allokation von Haplotyp-Blöcken, die kleiner als eine bestimmte Menge an genetischen Polymorphismen sind, der großelterliche Ursprung von Haplotyp-Blöcken, die größer als die genannte Menge an Polymorphismen sind, genutzt wird.

10. Die Methode gemäß einem der Ansprüche 8 oder 9, wobei das Neuzuordnen der Allel-Allokation von Haplotyp-Blöcken, die kleiner als eine bestimmte Menge an genetischen Polymorphismen sind, dadurch erfolgt, dass der großelterliche Ursprung der ersten linken und rechten flankierenden Haplotyp-Blöcke, die größer als der Schwellenwert sind, genutzt wird.

11. Die Methode gemäß einem der Ansprüche 8 bis 10, wobei Haplotyp-Blöcke, die in ihrer Menge an genetischen Polymorphismen kleiner als ein bestimmter Schwellenwert T₁ sind, dem ersten Haplotyp-Block auf der linken oder rechten Seite des Haplotyp-Blocks, der größer als der Schwellenwert T₁ ist, zugeordnet werden, nur wenn diese zwei Haplotyp-Blöcke demselben Großelternteil zugeordnet werden.

12. Die Methode gemäß Anspruch 1 oder 2, wobei das Neuzuordnen der Allel-Allokation eines oder mehrerer genetischer Polymorphismen und/oder Zwischenregionen auf Bildverarbeitungstechniken basiert.

13. Die Methode gemäß Anspruch 12, wobei die Bildverarbeitungstechnik die Anwendung eines "1D-Medianfilters" ist.

14. Die Methode gemäß einem der vorherigen Ansprüche, wobei die genetische Polymorphismus-Typisierung durch Einzelnukleotid-Polymorphismus-Typisierung durchgeführt wird.

15. Die Methode gemäß einem der vorherigen Ansprüche, wobei die genetische Polymorphismus-Typisierung durch DNA-Sequenzierung durchgeführt wird.

16. Die Methode gemäß einem der vorherigen Ansprüche, wobei die Einzelzelle eine menschliche oder tierische Blastomere ist.

## Revendications

1. Un procédé pour l'haplotypage d'une cellule unique comprenant les étapes suivantes :
a. Sélection d'une cellule unique ;
b. Lyse de cette cellule unique ;
c. Amplification de l'ADN de cette cellule unique ;
d. Typage massivement parallèle du polymorphisme génétique de l'ADN amplifié de cette cellule unique ;
e. Reconstruction de l'haplotype du génotype de la cellule unique à l'aide d'un logiciel d'haplotypage utilisant tous les polymorphismes génétiques ou une sélection de ceux-ci ;
f. Réaffectation de l'attribution allélique d'un ou plusieurs polymorphisme(s) génétique(s) et/ou région(s) intermédiaire(s), sur la base des informations des polymorphismes génétiques avoisinants.

2. Un procédé pour l'haplotypage de deux cellules comprenant les étapes suivantes :
a. Sélection de deux cellules ;
b. Lyse de ces deux cellules ;
c. Amplification de l'ADN cellulaire de chaque cellule ;
d. Typage massivement parallèle du polymorphisme génétique de l'ADN amplifié de chaque cellule ;
e. Production d'un génotype virtuel constitué de signaux du polymorphisme génétique concordants entre les deux cellules uniques génotypées séparément ;
f. Reconstruction de l'haplotype de ce génotype virtuel à l'aide d'un logiciel d'haplotypage ;
g. Réaffectation de l'attribution allélique d'un ou plusieurs polymorphisme(s) génétique(s) et/ou région(s) intermédiaire(s), sur la base des informations des polymorphismes génétiques avoisinants.

3. Le procédé suivant la revendication 1 ou la revendication 2, dans lequel la réaffectation de l'attribution allélique d'un ou plusieurs polymorphisme(s) génétique(s) et/ou région(s) intermédiaire(s) se fait via la réaffectation de l'attribution allélique des blocs haplotypiques inférieurs à une certaine quantité de polymorphismes génétiques.

4. Le procédé suivant la revendication 3, dans lequel la réaffectation de l'attribution allélique des blocs haplotypiques inférieurs à une certaine quantité de polymorphismes génétiques utilise l'origine grand-parentale de blocs haplotypiques supérieurs à cette quantité de polymorphismes génétiques.

5. Le procédé suivant l'une quelconque des revendications 3 à 4, dans lequel la réaffectation de l'attribution allélique des blocs haplotypiques inférieurs à une certaine quantité de polymorphismes génétiques se fait en utilisant l'origine grand-parentale des premiers blocs haplotypiques du flanc gauche et du flanc droit, qui sont supérieurs au seuil.

6. Le procédé suivant l'une quelconque des revendications 3 à 5, dans lequel les blocs haplotypiques qui sont plus petits, en termes de quantité de polymorphismes génétiques, qu'un certain seuil T₁, sont affectés aux premiers blocs haplotypiques du côté gauche ou droit du bloc haplotypique supérieur au seuil T₁, uniquement si ces deux blocs haplotypiques sont affectés au même grand-parent.

7. Un procédé visant à améliorer des haplotypes calculés dérivés d'une cellule unique ou de deux cellules, comprenant la réaffectation de l'attribution allélique d'un ou plusieurs polymorphisme(s) génétique(s) et/ou région(s) intermédiaire(s), sur la base des informations des polymorphismes génétiques avoisinants.

8. Le procédé suivant la revendication 7, dans lequel la réaffectation de l'attribution allélique d'un ou plusieurs des polymorphismes génétiques et/ou régions intermédiaires comprend la réaffectation de l'attribution allélique des blocs haplotypiques inférieurs à une certaine quantité de polymorphismes génétiques.

9. Le procédé suivant la revendication 8, dans lequel la réaffectation de l'attribution allélique des blocs haplotypiques inférieurs à une certaine quantité de polymorphismes génétiques utilise l'origine grand-parentale de blocs haplotypiques supérieurs à cette quantité de polymorphismes génétiques.

10. Le procédé suivant l'une quelconque des revendications 8 ou 9, dans lequel la réaffectation de l'attribution allélique des blocs haplotypiques inférieurs à une certaine quantité de polymorphismes génétiques se fait en utilisant l'origine grand-parentale des premiers blocs haplotypiques du flanc gauche et du flanc droit, qui sont supérieurs au seuil.

11. Le procédé suivant l'une quelconque des revendications 8 à 10, dans lequel les blocs haplotypiques qui sont inférieurs, en termes de quantité de polymorphismes génétiques, à un certain seuil T₁, sont affectés aux premiers blocs haplotypiques du côté gauche ou droit du bloc haplotypique supérieur au seuil T₁, uniquement si ces deux blocs haplotypiques sont affectés au même grand-parent.

12. Le procédé suivant la revendication 1 ou 2, dans lequel la réaffectation de l'attribution allélique d'un ou plusieurs polymorphisme(s) génétique(s) et/ou région(s) intermédiaire(s) est basée sur les techniques d'acquisition d'images.

13. Le procédé suivant la revendication 12, dans lequel la technique d'acquisition d'images est l'application d'un « filtre médian 1 D ».

14. Le procédé suivant l'une quelconque des revendications précédentes, dans lequel le typage du polymorphisme génétique est réalisé par typage du polymorphisme nucléotidique simple.

15. Le procédé suivant l'une quelconque des revendications précédentes, dans lequel le typage du polymorphisme génétique est réalisé par séquençage de l'ADN.

16. Le procédé suivant l'une quelconque des revendications précédentes, dans lequel la cellule unique est un blastomère humain ou animal.
